(19) **Europäisches Patentamt**
**European Patent Office**
**Office européen des brevets**

(11) **EP 3 170 252 B1**

(12) **FASCICULE DE BREVET EUROPEEN**

(45) Date de publication et mention de la délivrance du brevet:
**05.06.2019 Bulletin 2019/23**

(51) Int Cl.:
*H02M 7/10* (2006.01)    *H02J 7/32* (2006.01)
*H02J 7/34* (2006.01)    *H02N 2/00* (2006.01)

(21) Numéro de dépôt: **15752931.4**

(22) Date de dépôt: **17.07.2015**

(86) Numéro de dépôt international:
**PCT/EP2015/066502**

(87) Numéro de publication internationale:
**WO 2016/009087 (21.01.2016 Gazette 2016/03)**

(54) **DISPOSITIF ÉLECTRONIQUE AUTONOME À ALIMENTATION PAR TRANSDUCTION ÉLECTROSTATIQUE PRODUITE PAR UNE CAPACITÉ VARIABLE**

AUTONOME ELEKTRONISCHE VORRICHTUNG MIT VERSORGUNG DURCH ELEKTROSTATISCHE TRANSDUKTION, DIE VON EINEM DREHKONDENSATOR ERZEUGT WIRD

AUTONOMOUS ELECTRONIC DEVICE WITH SUPPLY BY ELECTROSTATIC TRANSDUCTION PRODUCED BY A VARIABLE CAPACITOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.07.2014 FR 1456987**

(43) Date de publication de la demande:
**24.05.2017 Bulletin 2017/21**

(73) Titulaires:
• **Université Paris-Sud**
  **91405 Orsay Cedex (FR)**
• **Centre National de la Recherche Scientifique**
  **75016 Paris (FR)**

(72) Inventeur: **LEFEUVRE, Elie**
  **F-93100 Montreuil (FR)**

(74) Mandataire: **IPAZ**
  **Parc Les Algorithmes, Bâtiment Platon**
  **CS 70003 Saint-Aubin**
  **91192 Gif-sur-Yvette Cedex (FR)**

(56) Documents cités:
**WO-A1-2013/059562    WO-A2-2007/102937**
**US-A1- 2010 298 720**

• **CHENLING HUANG ET AL: "Low-threshold voltage multipliers based on floating-gate charge-pumps", BIOMEDICAL CIRCUITS AND SYSTEMS CONFERENCE, 2008. BIOCAS 2008. IEEE, IEEE, PISCATAWAY, NJ, USA, 20 novembre 2008 (2008-11-20), pages 205-208, XP031398839, ISBN: 978-1-4244-2878-6**
• **JASON LEE WARDLAW ET AL: "Low-Power Circuits and Energy Harvesting for Structural Health Monitoring of Bridges", IEEE SENSORS JOURNAL, IEEE SERVICE CENTER, NEW YORK, NY, US, vol. 13, no. 2, 1 février 2013 (2013-02-01), pages 709-722, XP011487785, ISSN: 1530-437X, DOI: 10.1109/JSEN.2012.2226712**
• **DE QUEIROZ ANTONIO CARLOS M ET AL: "Analysis of the doubler of electricity considering a resistive load", 2013 IEEE 56TH INTERNATIONAL MIDWEST SYMPOSIUM ON CIRCUITS AND SYSTEMS (MWSCAS), IEEE, 4 août 2013 (2013-08-04), pages 45-48, XP032525686, ISSN: 1548-3746, DOI: 10.1109/MWSCAS.2013.6674581 ISBN: 978-1-4799-4134-6 [extrait le 2013-11-24]**

## Description

L'invention concerne un dispositif électronique de type fonctionnant sans apport d'énergie électrique extérieure et comprenant un circuit d'alimentation électrique comprenant au moins un condensateur variable, sous l'effet d'un mouvement mécanique alternatif, connecté à un dispositif de stockage par un circuit de redressement. Ce circuit d'alimentation comprend, sous forme de composants passifs et sans moyen de synchronisation :

- une branche de génération comprenant le condensateur variable et un condensateur de polarisation montés en série, laquelle branche est montée en parallèle avec le circuit de redressement et avec une branche de stockage, entre :

   ◦ un noeud dit de base à l'extrémité de la branche de génération du côté du condensateur variable,
   ◦ un noeud dit de sortie à l'extrémité de la branche de génération du côté du condensateur de polarisation ;

- une branche de retour de charge unidirectionnelle :

   ◦ vers la branche de génération en un noeud de polarisation situé entre le condensateur variable et le condensateur de polarisation ;
   ◦ depuis le redresseur, par au moins une extrémité recevant une partie de l'énergie électrique produite,

Selon l'invention, l'ensemble formé par ladite branche de génération, le circuit de redressement, la branche de stockage et la branche de retour de charge constitue un circuit électrique qui demeure inchangé et permanent pendant au moins une pluralité de cycles du condensateur variable.

Ce circuit réalise ainsi à chaque cycle un complément de charge du condensateur de polarisation selon un fonctionnement autosynchrone, c'est-à-dire qu'il ne nécessite aucun moyen de synchronisation par rapport aux mouvements mécaniques pendant ladite pluralité de cycles.

Le circuit d'alimentation comprend un multiplicateur de tension connecté au noeud de polarisation pour appliquer une tension multipliée par rapport à celle existant entre :

- le noeud de sortie, et
- le noeud de base ou l'une des extrémités de la branche de stockage.

L'invention concerne plus particulièrement un dispositif électronique prévu pour être implanté dans le corps humain ou animal in vivo sans conserver de liaison physique avec l'extérieur, et qui comprend ou forme un tel dispositif électronique.

Elle concerne en outre un circuit d'alimentation électrique pour former le circuit d'alimentation électrique d'un tel dispositif.

## Etat de la technique

Le domaine de l'invention est celui des dispositifs électroniques, ou comportant une partie électronique, qui doivent fonctionner de façon autonome pendant une période prolongée sans apport extérieur d'énergie électrique, bien plus longtemps que ne le permettrait la seule quantité d'énergie embarquée initialement.

De tels dispositifs électroniques peuvent être par exemple des dispositifs à basse consommation destinés à être implantés dans le corps humain ou animal, pour lesquels il est impossible ou complexe de prévoir une connexion électrique de rechargement. Il peut s'agir aussi par exemple des capteurs de mesure ou de détection communicants sans fil, destinés à être disséminés ou installés dans des zones étendues, par exemple des détecteurs de signal radio, ou des capteurs environnementaux en milieu naturel, ou tout dispositif pour lequel une très grande autonomie est recherchée, par exemple des dispositifs destinés à être portés sur soi comme un vêtement intelligent, ou embarqués dans un système non prévu pour les accueillir comme une balise de surveillance ou de localisation.

Pour un tel dispositif, surtout lorsque l'on recherche une certaine miniaturisation, il a été proposé de l'alimenter par une énergie électrique produite par un dispositif formant un condensateur électrique dont la capacité varie sous l'effet d'une énergie mécanique reçue par le dispositif lui-même. Il peut s'agir par exemple de récupérer l'énergie mécanique ambiante, telle que des vibrations, des déformations mécaniques variables, des variations de pression d'un fluide, etc.

Un tel mode de production convertit l'énergie mécanique en énergie électrique par un procédé de transduction dit électrostatique. D'un point de vue électrique, le transducteur électrostatique peut être vu comme une capacité variable. Ce mode de transduction passe par un circuit électronique de gestion d'énergie qui est miniaturisable, mais nécessite actuellement une électronique complexe pour gérer les cycles de charge et de décharge du transducteur.

Or, dans les dispositifs connus, le fonctionnement d'un tel circuit de gestion de l'énergie nécessite plusieurs dizaines de Volts et représente une consommation non négligeable, alors que les niveaux de puissance générés par les dispositifs électrostatiques sont très faibles, typiquement dans une gamme de puissance comprise entre quelques nanowatts et quelques centaines de microwatts.

Parmi les premiers circuits proposés fonctionnant à des niveaux de puissance de l'ordre du μW, un circuit de conditionnement contraint en charge à été proposé par Meninger et al. (2001) "Vibration-to-Electric Energy Conversion" in IEEE Transactions on Very Large Scale Integration (VLSI) Systems, vol.9, iss.1, pp.64-76, et un circuit de conditionnement contraint en tension a été proposé par Torres et al. (2006) 49th IEEE International Midwest Symposium on Circuits and Systems, MWSCAS '06, 6-9 Aug. 2006, pp.65-69.

Cependant, ces deux types de circuits sont composés d'interrupteurs synchronisés avec le mouvement mécanique du transducteur. Les interrupteurs nécessitent d'être commandés par un circuit électronique complémentaire très énergivore comparativement aux puissances/énergie générées. Il n'existe pour l'instant pas de mise en oeuvre viable de ce type de circuit pour des puissances faibles, par exemple inférieures à une centaine de microwatts.

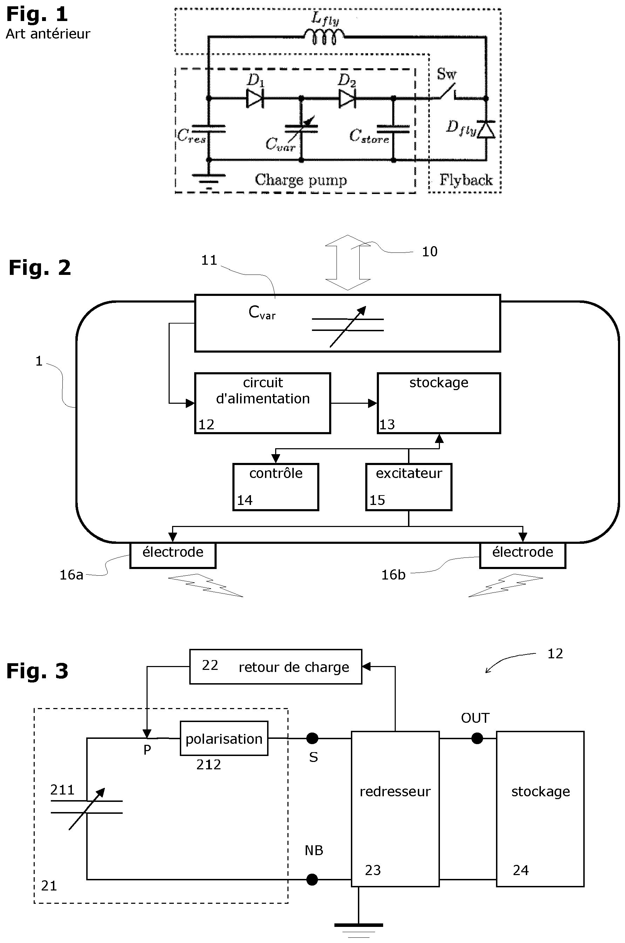

Comme illustré en FIGURE 1, un circuit est proposé par Bernard C. Yen, "A Variable-Capacitance Vibration-to-Electric Energy Harvester", IEEE Transactions On Circuits And Systems, Vol. 53(2), pp.288-295, 2005.

Ce circuit utilise les variations de capacité du condensateur variable $C_{var}$ grâce à l'utilisation d'une pompe de charge composée de diodes et de condensateurs. L'énergie électrique générée par la capacité est d'abord stockée dans la capacité $C_{store}$, puis périodiquement renvoyée dans $C_{res}$ à l'aide d'un circuit de retour par l'intermédiaire de l'inductance $L_{fly}$.

Ce circuit présente cependant des inconvénients. En particulier, le circuit de retour comporte une inductance qui induit des limitations en termes de miniaturisation du circuit.

De plus, il comporte aussi un interrupteur dont la commande doit être ajustée en fonction des conditions de fonctionnement du transducteur électrostatique, par exemple l'état de charge des condensateurs $C_{res}$ et $C_{store}$, ainsi que l'amplitude et la fréquence des vibrations, ceci de manière à maximiser la puissance générée. Cette commande est réalisée à l'aide d'un circuit intelligent, qui est aussi consommateur d'énergie.

WO 2013/059562 A1 divulgue un circuit d'alimentation avec comme source un condensateur variable, de faible puissance, sans multiplicateur de tension.

Un but de l'invention est de fournir un dispositif électronique capable de convertir une énergie mécanique ambiante en énergie électrique pour son alimentation, en sa totalité ou en tant que complément ou prolongation d'autonomie. L'invention cherche aussi à optimiser le rendement de cette conversion, la simplicité, la fiabilité et le coût de sa fabrication et de son fonctionnement.

## Exposé de l'invention

L'invention propose un dispositif électronique de type fonctionnant sans apport d'énergie électrique extérieure, de préférence au cours de sa vie ou au moins d'un cycle de fonctionnement. Ce fonctionnement peut se faire de façon autonome ou avec prolongation d'autonomie, par exemple plusieurs semaines à plusieurs années possiblement sans liaison matérielle/physique avec l'extérieur ou de façon autonome.

Ce dispositif comprend un circuit d'alimentation électrique agencé pour l'alimenter, de préférence en totalité, mais aussi en complément ou en prolongation d'une source embarquée. Ce circuit d'alimentation électrique typiquement agencé pour fournir une alimentation avec une tension stable, ou ne dépassant pas une tension maximale adaptée à un dispositif autonome, e.g. appareil embarqué de façon implantée ou transportable par un individu ou au sein d'un véhicule autonome en énergie. Il s'agit typiquement d'une basse tension (moins de 400V) voire d'une tension faible, par exemple inférieure à 48V voire inférieure à 12V, ou 6V ou 4V.

Cette alimentation se fait de préférence à travers un élément de stockage, par exemple un condensateur ou un supercondensateur ou une batterie électrochimique rechargeable. Elle peut aussi se faire à travers un élément de consommation d'énergie électrique, par exemple directement la charge consommatrice formée par le fonctionnement du dispositif.

Ce circuit d'alimentation électrique comprend au moins un composant, dit condensateur variable, présentant une capacité électrique variable sous l'effet d'un mouvement mécanique alternatif. Un tel mouvement peut être qualifié d'oscillatoire dans la mesure où il comprend un déplacement entre plusieurs positions extrêmes, même si ce mouvement ne présente pas forcément un caractère de régularité systématique.

Un tel mouvement est typiquement en plusieurs phases, se succédant de façon alternative, sans continuité de direction (ou de rotation) entre ces différentes phases. Par exemple, ce mouvement peut être celui produit par un mécanisme ou un organe d'entraînement par exemple les battements du coeur ou d'un autre muscle.

Il peut s'agir d'un ou plusieurs composants, chacun formant à lui seul une capacité variable c'est-à-dire capable à lui seul d'augmenter la charge.

Un point important de l'invention est qu'elle permet d'utiliser, et propose préférentiellement de le faire, un tel condensateur variable formé d'une capacité variable, ou de plusieurs capacités variables qui fonctionnent dans le même sens. C'est-à-dire que, lorsqu'il y en a plusieurs, elles varient toutes dans le même sens à chaque instant, et sont de préférence mécaniquement solidaires entre elles. Cette caractéristique permet une grande compacité et simplicité dans l'architecture de cette ou ces capacités variables, et dans leur intégration au sein du dispositif électronique ainsi alimenté.

Ce condensateur variable :

- est agencé pour recevoir ledit mouvement mécanique de la part de l'environnement dudit dispositif électronique, par exemple par inertie, déplacement, ou compression, et
- est connecté audit élément de stockage au moyen d'un circuit de redressement pour augmenter l'énergie électrique qui y est stockée.

Selon l'invention, ce circuit d'alimentation électrique comprend une branche de génération formée par au moins le condensateur variable et un condensateur de polarisation (de préférence à capacité fixe) montés en série. Cette branche de génération est montée en parallèle avec le circuit de redressement et avec une branche de stockage comprenant l'élément de stockage. Elle est montée entre :

◦ un noeud dit de base qui est situé à l'extrémité de la branche de génération du côté du condensateur variable et est maintenu à un potentiel de référence (de façon permanente ou d'un cycle à l'autre),
◦ un noeud dit de sortie situé à l'extrémité de la branche de génération du côté du condensateur de polarisation ;

Ainsi, le potentiel entre le condensateur variable et le condensateur de polarisation présente une valeur moyenne qui varie lorsque la charge augmente dans l'élément de stockage.

Selon l'invention, ce circuit d'alimentation électrique comprend en outre une branche de retour de charge d'un type apte à conduire un courant électrique unidirectionnel, par exemple sous la forme d'une ou plusieurs diodes. Cette branche de retour de charge est ainsi conductrice :

◦ depuis le circuit de redressement, par au moins une deuxième extrémité recevant une partie de l'énergie électrique produite par ledit circuit d'alimentation,
◦ vers la branche de génération, par au moins une première extrémité connectée à un noeud de la branche de génération qui est situé entre le condensateur variable et le condensateur de polarisation, c'est-à-dire à un noeud commun ou connecté à ces deux éléments, et est ici appelé noeud de polarisation.

En outre, selon l'invention, un tel circuit d'alimentation électrique comprend un circuit agencé pour former un circuit multiplicateur de tension qui est connecté à la branche de génération en un noeud de polarisation situé entre le condensateur variable et le condensateur de polarisation, pour y appliquer une tension multipliée par rapport à la tension existant entre

- d'une part le noeud de sortie de ladite branche de génération, et
- d'autre part au noeud de base ou à l'une des extrémités de la branche de stockage.

Selon l'invention, ladite branche de génération, le circuit de redressement, la branche de stockage et la branche de retour de charge forment un ensemble qui constitue un circuit électrique, dit circuit passif, dont le comportement est passif et qui demeure inchangé et permanent dans ses caractéristiques pendant au moins une pluralité de cycles du condensateur variable, de préférence un nombre de cycles supérieur d'un facteur 100 voire 1000 ou 10000. C'est-à-dire que, pendant cette durée, cet ensemble ne présente pas d'interruption, ni de modifications dans le temps des caractéristiques de ces quatre éléments. C'est-à-dire que ces éléments (y compris le circuit multiplicateur) forment un circuit qui n'est pas modifié depuis l'extérieur pendant une durée prolongée, en dehors du mouvement imprimé au condensateur variable et de l'énergie fournie par la branche de stockage aux consommateurs qu'elle alimente.

Ainsi, au cours d'une durée "passive" couvrant plusieurs cycles, les composants de ce circuit sont inchangés : ils se comportent de façon identique d'un cycle à l'autre, c'est à dire qu'ils réagissent aux conditions internes au circuit selon les mêmes lois et avec les mêmes caractéristiques. De préférence, durant une telle durée, ce circuit spécifique ne communique avec l'extérieur que par le mouvement qu'il reçoit et l'énergie qu'il fournit, et est isolé du reste du monde extérieur.

Ce circuit passif ne reçoit pas de commandes modifiant sa structure pendant cette durée passive, comme pourrait le faire par exemple une commande de changement d'état d'un interrupteur, ou un signal de blocage ou de mise en conduction d'un transistor. Au cours de cette durée couvrant de plusieurs cycles, les composants de ce circuit sont inchangés : ils se comportent de façon identique d'un cycle à l'autre, c'est à dire qu'ils réagissent aux conditions internes au circuit selon les mêmes lois et avec les mêmes caractéristiques. Durant une telle durée, ce circuit spécifique ne communique avec l'extérieur que par le mouvement qu'il reçoit et l'énergie qu'il fournit, et est isolé du reste du monde extérieur. Il s'agit donc bien d'un circuit autonome, et ce de façon stable sur une durée significative.

Par exemple ce circuit passif ne comprend pas de composants actifs à entrée de commande tels que des transistors ; ou s'il en comprend, ces derniers sont commandés avec une période plus grande que ladite pluralité de cycles, par exemple pour un réglage ou un ajustement de temps en temps, par exemple quotidien ou mensuel dans le cas d'un stimulateur cardiaque à environ 100 cycles par minute. En particulier, l'ensemble formé par ladite branche de génération, le circuit de redressement, la branche de stockage et la branche de retour de charge ne comporte aucun composant magnétique, c'est-à-dire aucun composant de type inductance, inductance couplée ou transformateur magnétique.

Ainsi, à chaque à chaque cycle, le circuit d'alimentation réalise un complément de charge du condensateur de polarisation selon un mode de fonctionnement qui est autosynchrone, c'est-à-dire qu'il ne nécessite aucun moyen de synchronisation par rapport aux mouvements mécaniques pendant ladite pluralité de cycles.

Ce fonctionnement est ainsi complètement autosynchrone, quelle que soit la fréquence d'oscillation et sans nécessiter de pilotage d'un interrupteur ou un transistor, au moins sur toute cette durée "passive".

Optionnellement, tout ou partie des composants mentionnés ici comme étant des diodes sont remplacé chacun par un sous-circuit réalisant une "fonction diode", de préférence à base de composants entièrement passifs et par exemple uniquement avec des diodes, des transistors et des condensateurs. Un tel sous-circuit à "fonction diode" est par exemple une première diode sur laquelle est branché en parallèle un transistor (par exemple par drain et source), qui vient la court-circuiter lorsque sa commande (grille) est activée par la sortie d'une deuxième diode qui conduit depuis la sortie de la première diode vers un condensateur relié à la masse. Un tel sous-circuit permet de remplir la fonction d'une diode sans présenter le seuil de tension d'une diode simple. Le transistor étant commandé directement par la conduction de la diode, sans aucun signal extérieur, un tel sous-circuit se comporte comme une diode simple et reste conforme au fonctionnement autonome et autosynchrone du circuit d'alimentation pendant la durée dite "passive"

## Modes de réalisation avec multiplicateur de tension

Selon une particularité, l'invention propose en outre d'intégrer un circuit de type multiplicateur de tension au sein du circuit d'alimentation électrique du dispositif. Ce circuit d'alimentation électrique comprend ainsi un circuit multiplicateur de tension qui est connecté à la branche de génération en un noeud de polarisation situé entre le condensateur variable et le condensateur de polarisation. Ce multiplicateur est connecté et configuré pour à ce noeud de polarisation une tension multipliée par rapport à la tension existant entre :

- d'une part le noeud de sortie de ladite branche de génération, et
- d'autre part le noeud de base ou l'une des extrémités de la branche de stockage.

Une telle utilisation d'un multiplicateur de tension est particulièrement intéressante pour améliorer la capacité d'énergie convertie à chaque cycle, en particulier lorsque le condensateur variable présente une faible variation de sa capacité.

Les multiplicateurs de tension sont connus pour produire une forte élévation de tension à partir d'une source classique de courant alternatif sans contrainte de quantité d'énergie, typiquement à partir d'un réseau de distribution de courant alternatif en 110V ou 220V.

A l'inverse de ces usages connus, le multiplicateur pallie dans l'invention la faible variation du transducteur variable, ce qui constitue un problème majeur des alimentations autonomes à base transducteur électromécanique ; en particulier dans les types d'appareils envisagés ici et plus particulièrement dans les appareils à forte miniaturisation et/ou faible débattement mécanique.

Selon une particularité, la branche de retour de charge comprend une diode orientée depuis le redresseur vers la branche de génération, ou plusieurs diodes montées dans le même sens, et de préférence en série.

Plus particulièrement, le circuit d'alimentation électrique, ou au moins un ou plusieurs des circuits parmi la branche de génération, la branche de stockage, la branche de retour de charge et le circuit de redressement, et le circuit multiplicateur, est réalisé (de préférence sous la forme d'un circuit intégré unique) de façon à ne comprendre que des composants passifs et des semi-conducteurs non commandés, et en particulier uniquement des diodes et des condensateurs (en plus des conducteurs qui les relient et éventuellement de résistances ohmiques).

Le condensateur variable peut utiliser tout type de technologie, y compris des polymères électroactifs dont les caractéristiques capacitives varient avec leurs déformations, tels que ceux utilisés pour réaliser des muscles artificiels.

Cependant, l'invention est particulièrement intéressante à mettre en oeuvre avec un ou des condensateurs variables à fluide. Il peut s'agir par exemple de condensateurs variables à air fonctionnant par mouvement relatif des électrodes. Il peut s'agir aussi de condensateurs variables fonctionnant par un déplacement de liquide, par exemple produisant directement un déplacement du matériau diélectrique, ou produisant un déplacement relatif des électrodes. En effet, ces types de condensateurs variables sont particulièrement adaptés pour récupérer une énergie mécanique de faible puissance et/ou de faible course, ce qui est particulièrement intéressant pour récupérer une énergie d'origine biologique, par exemple pour un appareil implantable intracorporel comme un appareil médical de surveillance et/ou de stimulation.

Selon une particularité, l'invention porte sur un appareil autonome de faible puissance, par exemple inférieure à un watt voire à 500mW ou 100mW.

Selon une autre particularité de l'invention, il est proposé un dispositif électronique prévu pour être implanté dans le corps humain ou animal in vivo sans conserver de liaison physique avec l'extérieur, caractérisé en ce qu'il comprend ou forme un dispositif électronique tel qu'énoncé ici.

Selon un autre aspect de l'invention, il est proposé un circuit d'alimentation électrique pour un dispositif électronique tel qu'énoncé ici.

D'autres particularités sont prévues et énoncées dans la suite de la présente description. Des modes de réalisation variés de l'invention sont prévus, intégrant selon l'ensemble de leurs combinaisons possibles les différentes caractéristiques optionnelles exposées ici.

## Liste des figures

D'autres particularités et avantages de l'invention ressortiront de la description détaillée d'un mode de mise en oeuvre nullement limitatif, et des dessins annexés sur lesquels :

- la FIGURE 1 est un schéma illustrant un circuit d'alimentation électrique à partir d'un condensateur variable selon l'art antérieur,
- la FIGURE 2 est un schéma de principe d'un dispositif selon un exemple de mode de réalisation de l'invention, appliqué à un stimulateur cardiaque ;
- la FIGURE 3 est un schéma de principe du circuit d'alimentation électrique d'un dispositif dans un exemple de mode de réalisation selon l'invention ;
- la FIGURE 4 est un schéma illustrant le circuit d'alimentation d'un dispositif, sans circuit de multiplication de tension, formant un circuit de base auquel peut s'appliquer l'invention ;
- les FIGURE 5a à FIGURE 5c sont des chronogrammes illustrant le cycle de fonctionnement du circuit d'alimentation de la FIGURE 4, avec :

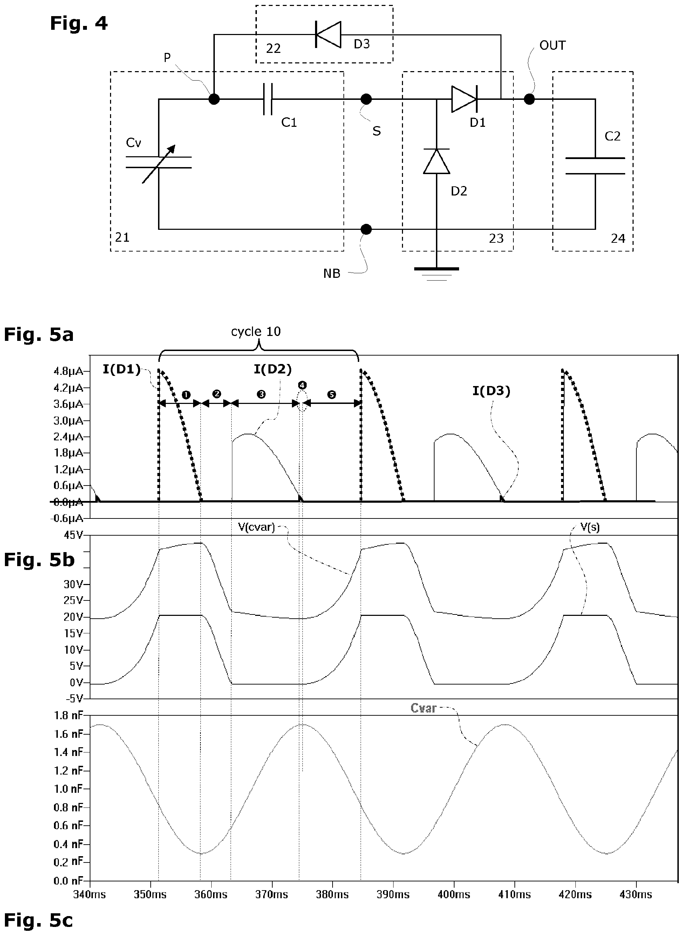

  - en FIGURE 5a, les intensités dans les trois diodes du circuit, et
  - en FIGURE 5b, les tensions aux bornes de la capacité variable et aux bornes de la branche de génération,
  - en FIGURE 5c, la valeur de la capacité du condensateur variable ;

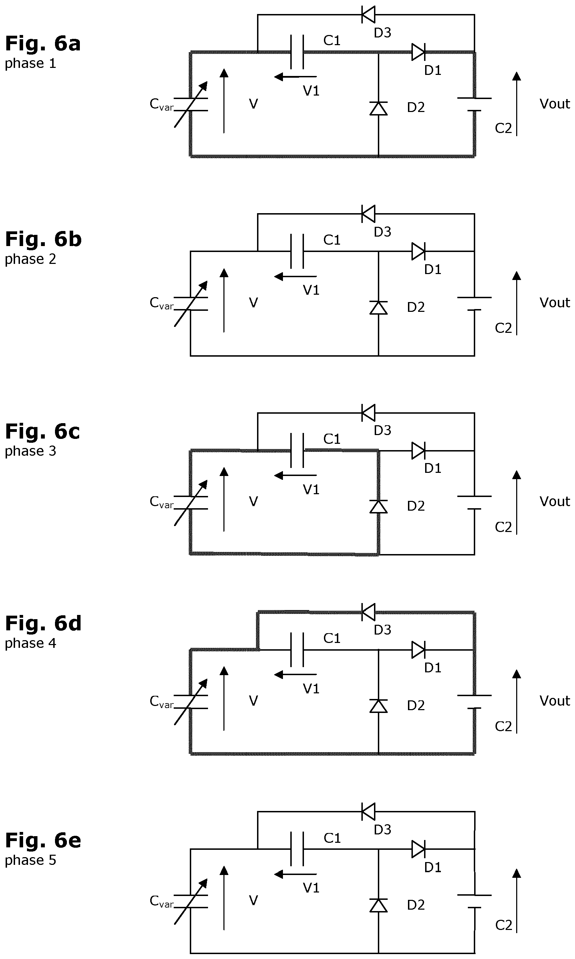

- les FIGURE 6a à FIGURE 6e sont des schémas du circuit de la FIGURE 4, illustrant les courants parcourant le circuit lors du cycle de fonctionnement ;

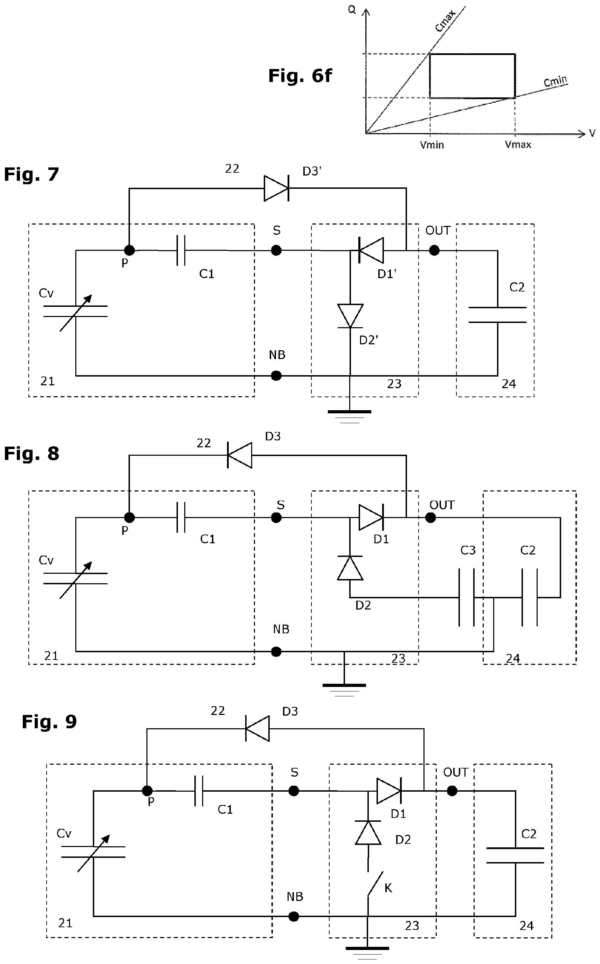

- la FIGURE 6f est un schéma illustrant le cycle de conversion d'énergie dans le plan charge-tension ;
- la FIGURE 7 est un schéma illustrant le circuit d'alimentation d'un dispositif formant une version inversée du circuit de base de la FIGURE 4 ;
- la FIGURE 8 est un schéma illustrant le circuit d'alimentation d'un dispositif réalisé à partir du circuit de base et dans lequel la capacité de stockage est d'un type à point milieu branché sur le redresseur ;
- la FIGURE 9 est un schéma illustrant le circuit d'alimentation d'un dispositif comprenant le circuit de base muni d'un interrupteur ;

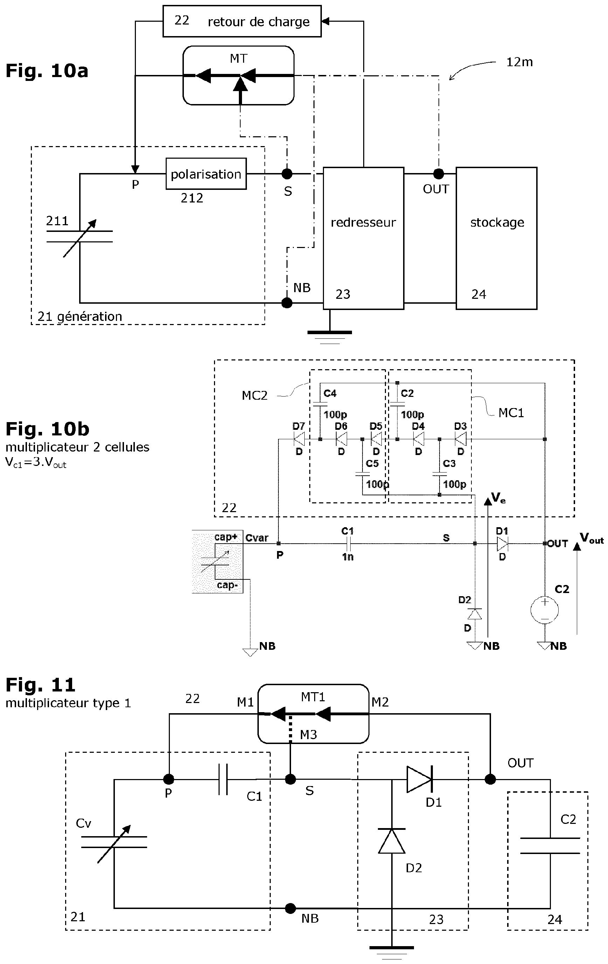

- la FIGURE 10a est un schéma illustrant le circuit d'alimentation d'un dispositif selon l'invention, dans un exemple de mode de réalisation comprenant un multiplicateur de tension ;
- la FIGURE 10b illustre le mode de réalisation à multiplicateur de tension de la FIGURE 10a, dans un exemple particulier à deux cellules ;
- la FIGURE 11 est un schéma de principe illustrant le circuit d'alimentation d'un dispositif selon l'invention, dans un cinquième mode de réalisation réalisé à partir du circuit de base dans une famille de versions selon une variante appelée **type 1** comprenant un circuit multiplicateur de tension intégré dans la branche de retour de charge depuis le dispositif de stockage vers la capacité variable ;

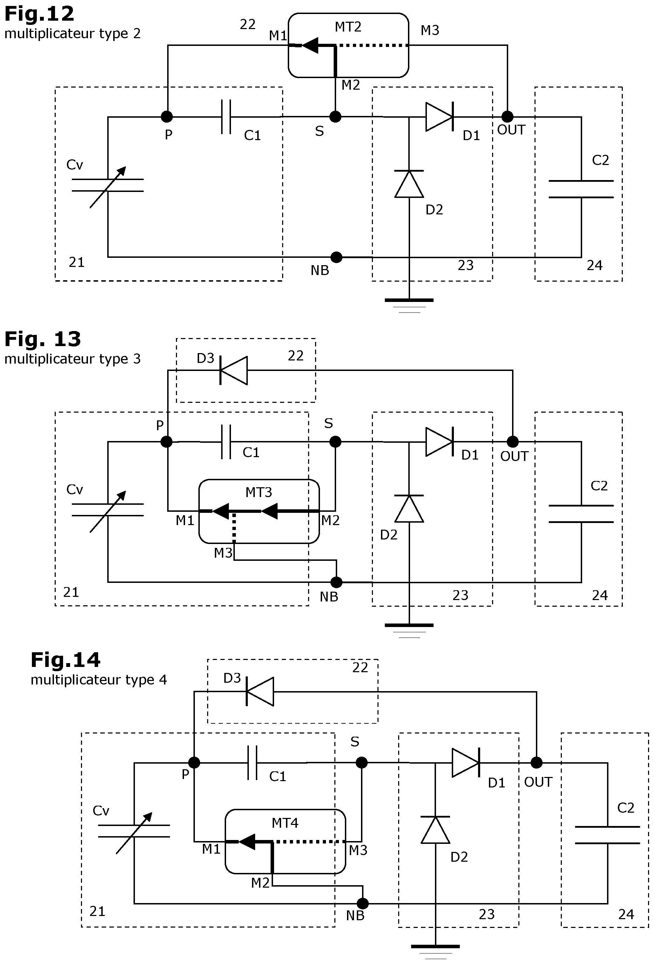

- la FIGURE 12 est un schéma de principe illustrant le circuit d'alimentation d'un dispositif selon l'invention, dans un sixième mode de réalisation réalisé à partir du circuit de base dans une famille de versions selon une variante appelée **type 2** comprenant un circuit multiplicateur de tension intégré dans la branche de retour de charge depuis le noeud de sortie vers la capacité variable ;
- la FIGURE 13 est un schéma de principe illustrant le circuit d'alimentation d'un dispositif selon l'invention, dans un septième mode de réalisation réalisé à partir du circuit de base dans une famille de versions selon une variante appelée **type 3** comprenant un circuit multiplicateur de tension intégré dans la branche de génération depuis le noeud de sortie vers la capacité variable ;

- la FIGURE 14 est un schéma de principe illustrant le circuit d'alimentation d'un dispositif selon l'invention, dans un huitième mode de réalisation réalisé à partir du circuit de base dans une famille de versions selon une variante appelée **type 4** comprenant un circuit multiplicateur de tension intégré dans la branche de génération depuis le noeud de base vers la capacité variable ;

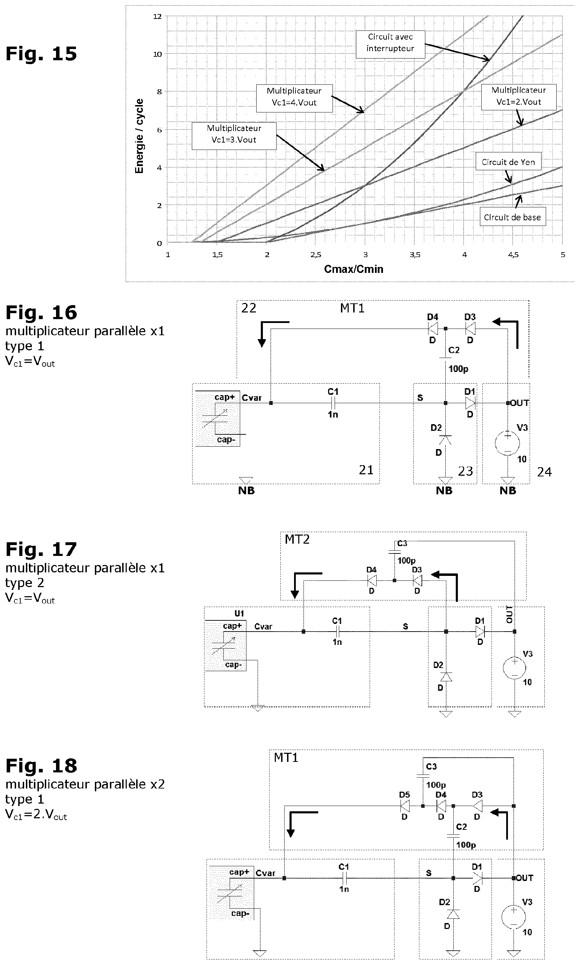

- la FIGURE 15 est un graphique représentant les performances d'énergie par cycle, obtenues par simulation pour différents modes de réalisation du circuit d'alimentation électrique d'un dispositif selon l'invention, en comparaison entre eux et avec le circuit de la FIGURE 1 ;

- les FIGURE 16 et suivantes sont des schémas représentant le circuit d'alimentation d'un dispositif selon l'invention de l'un des modes de réalisation avec multiplicateur, dans des versions avec :

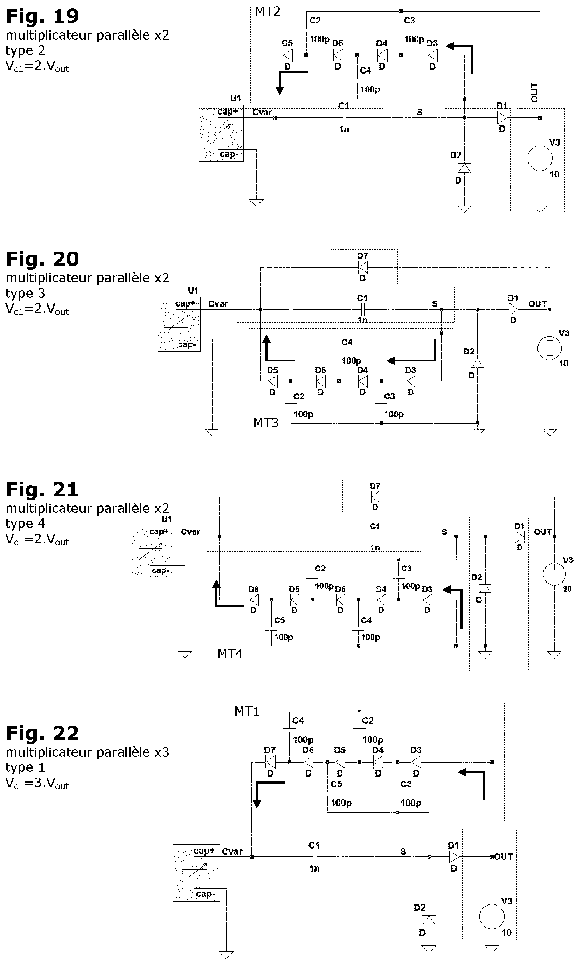

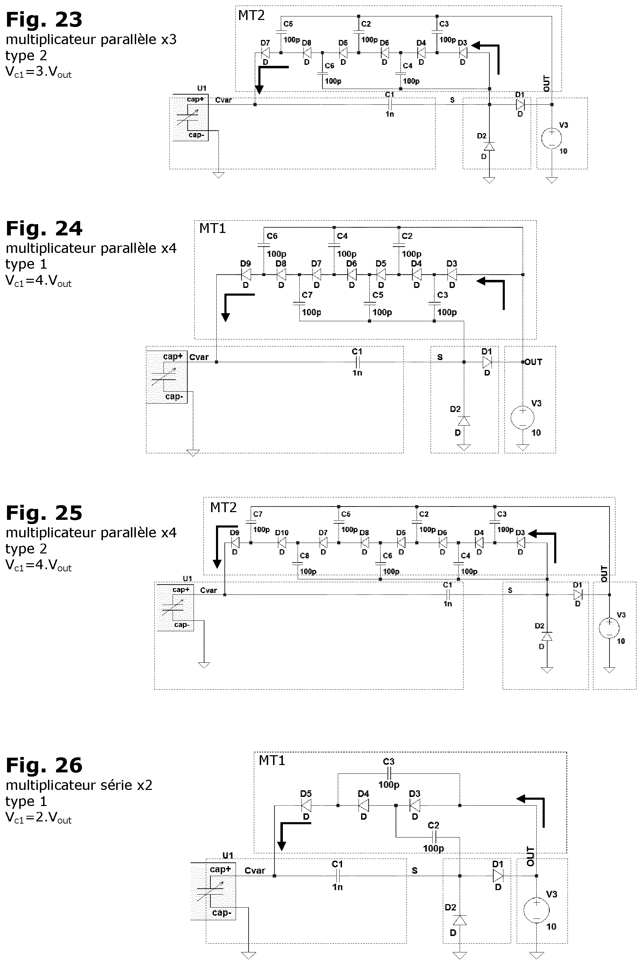

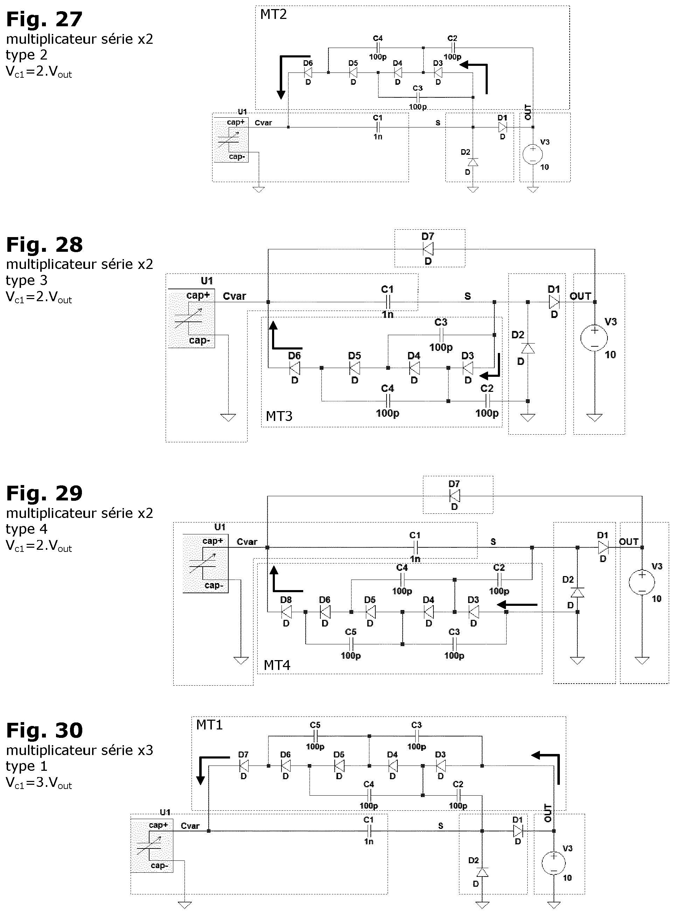

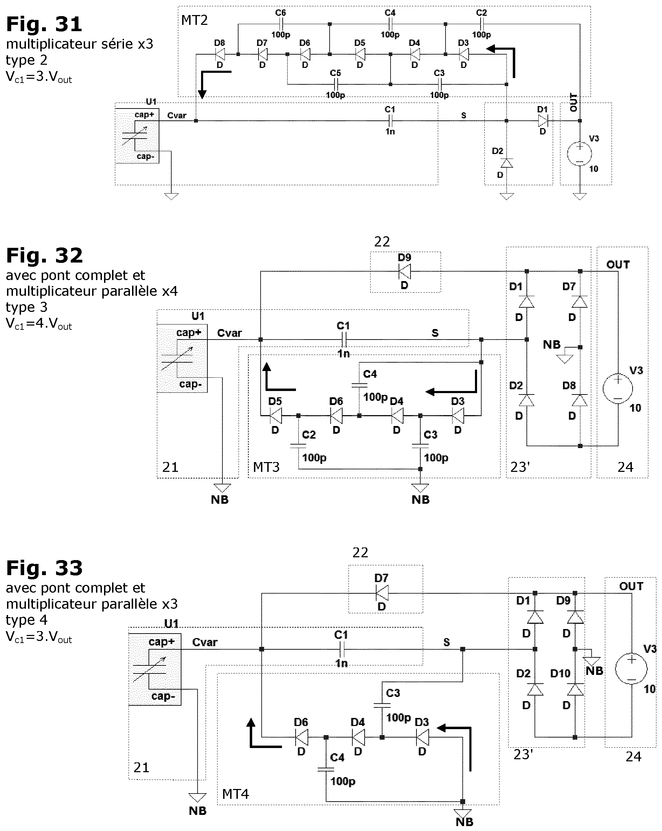

  ◦ en FIGURE 16, un multiplicateur de tension de **type 1** de type parallèle et d'un facteur 1 ;
  ◦ en FIGURE 17, un multiplicateur de tension de **type 2** de type parallèle et d'un facteur 1 ;
  ◦ en FIGURE 18, un multiplicateur de tension de **type 1** de type parallèle et d'un facteur 2 ;
  ◦ en FIGURE 19, un multiplicateur de tension de **type 2** de type parallèle et d'un facteur 2 ;
  ◦ en FIGURE 20, un multiplicateur de tension de **type 3** de type parallèle et d'un facteur 2 ;
  ◦ en FIGURE 21, un multiplicateur de tension de **type 4** de type parallèle et d'un facteur 2 ;
  ◦ en FIGURE 22, un multiplicateur de tension de **type 1** de type parallèle et d'un facteur 3, identique à celui de la FIGURE 10b ;
  ◦ en FIGURE 23, un multiplicateur de tension de **type 2** de type parallèle et d'un facteur 3 ;
  ◦ en FIGURE 24, un multiplicateur de tension de **type 1** de type parallèle et d'un facteur 4 ;
  ◦ en FIGURE 25, un multiplicateur de tension de **type 2** de type parallèle et d'un facteur 4 ;
  ◦ en FIGURE 26, un multiplicateur de tension de **type 1** de type série et d'un facteur 2 ;
  ◦ en FIGURE 27, un multiplicateur de tension de **type 2** de type série et d'un facteur 2 ;
  ◦ en FIGURE 28, un multiplicateur de tension de **type 3** de type série et d'un facteur 2 ;
  ◦ en FIGURE 29, un multiplicateur de tension de **type 4** de type série et d'un facteur 2 ;
  ◦ en FIGURE 30, un multiplicateur de tension de **type 1** de type série et d'un facteur 3 ;
  ◦ en FIGURE 31, un multiplicateur de tension de **type 2** de type série et d'un facteur 3 ;

- les FIGURE 32 et FIGURE 33 sont des schémas représentant le circuit d'alimentation d'un dispositif selon l'invention de l'un des modes de réalisation avec multiplicateur, dans des versions avec un circuit redresseur formé par un pont "complet" à 4 diodes, et :

  ◦ en FIGURE 32, un multiplicateur de tension de **type 3** de type parallèle et d'un facteur 4 ;
  ◦ en FIGURE 33, un multiplicateur de tension de **type 4** de type série et d'un facteur 3.

## Description d'exemples de modes de réalisation

La FIGURE 2 est un schéma de principe d'un dispositif selon un exemple de mode de réalisation de l'invention, appliqué à un stimulateur cardiaque 1. Une capacité variable 11 est actionnée par les battements du coeur, et est connectée au circuit d'alimentation 12, qui augmente la charge d'un élément de stockage 13 qui peut être par exemple une capacité ou un accumulateur chimique. Cette énergie est utilisée par un circuit d'excitation 15 pour alimenter des électrodes 16a et 16b qui stimulent le coeur en cas de besoin ou selon un rythme calculé par un circuit 14 de contrôle, par exemple relié à des moyens de détection non représentés ici.

L'invention propose d'utiliser une famille de circuits d'interface de génération, c'est-à-dire d'alimentation électrique, dont la structure peut être définie de la manière suivante, en référence à la FIGURE 3.

Le condensateur variable 211 est placé en série avec un composant de polarisation 212, par exemple un condensateur ou un supercondensateur ou un accumulateur d'énergie électrique.

Le dipôle ainsi formé constitue une branche de génération 21, qui est reliée à l'entrée d'un circuit redresseur 23, par exemple composé de diodes ou d'autres composants électroniques actifs réalisant la même fonction.

La sortie du circuit redresseur 23 est reliée à un composant de stockage 24 d'énergie électrique, c'est-à-dire par exemple un condensateur, un supercondensateur, un accumulateur d'énergie électrique.

Un circuit de retour charge 22 permet de maintenir une tension aux bornes du composant de polarisation 212.

Le composant de polarisation 212 se distingue du composant de stockage d'énergie électrique 24 en particulier en ce que l'énergie stockée dans le premier peut être très faible par rapport à celle stockée dans le deuxième. De préférence, l'énergie stockée dans le composant de polarisation 212 est au moins dix fois supérieure à celle générée par la capacité variable 211 au cours d'un cycle de conversion.

Mode de réalisation dit "circuit de base"

En FIGURE 4 est illustré le circuit d'alimentation d'un dispositif sans circuit de multiplication de tension, ici appelé "circuit de base" et auquel s'applique l'invention.

Dans ce mode de réalisation, le circuit d'alimentation comprend les éléments suivants, et de préférence uniquement ceux-ci :

- une branche de génération 21 comprenant le condensateur variable Cv et un ou plusieurs condensateur de polarisation C1, et de préférence uniquement ceux-ci (et de préférence en un seul exemplaire). Cette branche de génération est reliée par son noeud de base NB à un potentiel de référence, ici la masse, par un noeud qui est commun au redresseur 23 et à la branche de stockage 24 ;
- un circuit de redressement 23 comprenant les composants suivants, et de préférence uniquement ceux-ci :

   ∘ une diode D2 (de préférence unique), ou plusieurs diodes montées dans le même sens (de préférence en série), depuis le noeud de base NB et vers l'extrémité S de la branche de génération qui est située du côté du condensateur de polarisation C1,
   ∘ une diode D1 (de préférence unique), ou plusieurs diodes montées dans le même sens (de préférence en série), depuis l'extrémité S de la branche de génération 21 située du côté du condensateur de polarisation C1 et vers l'extrémité OUT de la branche de stockage 24 qui située du côté opposé au noeud de base NB ;

- une branche de stockage 24 comprenant un élément de stockage, et de préférence uniquement celui-ci, ledit élément de stockage comprenant un condensateur C2 ou un élément de stockage chimique (et de préférence uniquement l'un d'eux) ; et
- une branche de retour de charge 22 comprenant une diode D3 (de préférence unique), ou plusieurs diodes montées dans le même sens (de préférence en série), depuis l'extrémité OUT de la branche de stockage 24 située du côté opposé au noeud de base NB et vers le noeud de polarisation P situé dans la branche de génération 21 entre le condensateur variable Cv et le condensateur de polarisation C1.

Le redressement est réalisé par les diodes D1 et D2, formant un "demi-pont", tandis que la charge du condensateur de polarisation est assurée par D2 et D3. On notera que D2 est utilisée à la fois dans la fonction redressement et la fonction polarisation.

De préférence, les composants sont choisis comme suit : Condensateur de polarisation : $C1 > 2.(C_{MAX} - C_{MIN})$ voire $C1 > 5.(C_{MAX} - C_{MIN})$
et par exemple capacité C1 comprise entre 2 et 10 fois $(C_{MAX} - C_{MIN})$. Condensateur de stockage (si ce n'est pas une batterie) : $C_{OUT} > 3.C1$
et par exemple capacité C2 valant entre 10 fois et 1 million de fois $(C_{MAX} - C_{MIN})$
Diodes : toutes diodes à très faibles fuites et à très faibles capacités parasites.
Les diodes sont choisies de préférences avec un courant de fuite inférieur à cinq picoampères, et par exemple avec une tension de claquage entre 50 et 500V, par exemple de l'ordre de 100V.

Les FIGURE 5a à FIGURE 5c sont des chronogrammes illustrant le cycle de fonctionnement du circuit d'alimentation de la FIGURE 4, pour des composants présentant les valeurs suivantes :

- D1, D2, D3 : diodes à faible courant de fuite $I_{RMAX} = 5pA$ (selon la référence des composants PAD5 ou JPAD5)
- Cvar : valeur maximale de $Cvar_{MAX} = 1700$ pF, et valeur minimale $Cvar_{MIN} = 300pF$
- C1 : 10 nF

Pour un fonctionnement à 30 Hz et $V_{OUT} = 20V$, la puissance moyenne générée est de 11,5μW.

En FIGURE 5a sont représentées les formes d'ondes typiques des intensités ID1, ID2 et respectivement ID3, qui circulent dans les trois diodes du circuit D1, D2 et respectivement D3.

En FIGURE 5b sont représentées les tensions V(cvar) et respectivement V(s), qui sont présentent aux bornes de la capacité variable Cv et respectivement aux bornes de la branche de génération, 21 c'est-à-dire entre le noeud de base NB et le noeud de sortie S.

En FIGURE 5c sont représentées les formes d'ondes de la variation de la capacité du condensateur variable au cours du cycle mécanique 10.

Les FIGURE 6a à FIGURE 6e sont des schémas du circuit de la FIGURE 4, illustrant les courants parcourant le circuit lors des différentes phases 1 à 5 du cycle de fonctionnement qui sont indiquées en FIGURE 5.

En FIGURE 6a est illustrée la phase 1 : fin de montée de la tension V(cvar), et plateau de la tension V(s). La capacité variable diminue sous l'effet mécanique qui lui est appliqué, ce qui crée un courant ID1 au travers de la diode D1 et charge ainsi le condensateur de stockage C2.

En FIGURE 6b est illustrée la phase 2 : descente de la tension V(cvar) et de la tension V(s). La capacité variable augmente sous l'effet mécanique qui lui est appliqué, mais le circuit n'est parcouru par aucun courant. En effet, la tension V(s) étant inférieure à V(OUT) tout en étant strictement positive, les diodes D1 et D2 sont à l'état bloqué. De plus, la tension V(cvar) est supérieure à V(OUT), ce qui maintient la diode D3 à l'état bloqué.

En FIGURE 6c est illustrée la phase 3 : fin de descente de la tension V(cvar), et plateau de la tension V(s). La capacité variable continue à augmenter sous l'effet mécanique qui lui est appliqué, ce qui crée un courant ID2 au travers de la diode D2 et charge ainsi le condensateur de polarisation C1.

Ainsi qu'on le voit, la charge de repolarisation de C1 est principalement crée par la capacité variable Cvar, et n'est que très peu fournie par le condensateur de stockage C2. On obtient ainsi une utilisation performante de la capacité variable, et de faibles pertes dans l'énergie stockée dans C2.

En FIGURE 6d est illustré la phase 4 : La capacité variable arrive à son maximum sous l'effet mécanique qui lui est appliqué. Un courant ID3 passe très brièvement au travers de la diode D3. En fonctionnement permanent, la mise en conduction de D3 permet ainsi d'effectuer un complément de charge du condensateur de polarisation C1, pendant une très courte période.

En début de fonctionnement, cette phase permet le chargement initial du condensateur de polarisation C1, si besoin en puisant dans le condensateur de stockage C2, permettant d'amorcer le système de génération.

En FIGURE 6e est illustrée la phase 5 : La capacité variable diminue sous l'effet mécanique qui lui est appliqué, mais le circuit n'est parcouru par aucun courant. En effet, la tension V(s) étant strictement positive tout en étant inférieure à V(OUT), les diodes D1 et D2 sont à l'état bloqué. De plus, V(cvar) est supérieure à V(OUT), ce qui maintient D3 à l'état bloqué. Les tensions V(cvar) et tension V(s) augmentent et de l'énergie électrique est ainsi emmagasinée dans la capacité variable Cvar.

En FIGURE 6f est illustré le cycle de conversion d'énergie dans un graphique représentant le plan charge-tension.

Le cycle charge-tension réalisé au niveau du condensateur variable par ce circuit d'interface est un cycle rectangulaire compris entre les droites de pente Cmin et Cmax, capacités minimales et maximales du condensateur variable Cv.

Dans cette figure, l'aire du cycle est l'énergie convertie par période de variation du condensateur variable :

$$W = (Vmax - Vmin).(Cmax.Vmin - Cmin . Vmax) \qquad (1)$$

On note Vout la tension de sortie de l'interface, c'est à dire la tension $Vc_{res}$ pour le circuit de la FIGURE 1.

Dans le cas du circuit de la FIGURE 1, la tension Vmax conduit à une conversion d'énergie optimale lorsqu'elle vérifie (cf. thèse d'Andrii Dudka (2014)) :

$$V\max = \frac{1}{2}Vout\left(\frac{C\max}{C\min} - 1\right) \qquad (2)$$

L'énergie maximale convertie est alors :

$$W\max = \frac{1}{4}Vout^2.C\min\left(\frac{C\max}{C\min} - 1\right)^2 \qquad (3)$$

Dans l'invention, pour le circuit de base illustré en FIGURE 4, la tension Vmax est deux fois plus élevée que Vout et la tension Vmin est égale à Vout. L'énergie convertie par cycle s'exprime donc de la manière suivante :

$$W\max = Vout^2.C\min\left(\frac{C\max}{C\min} - 2\right) \qquad (4)$$

Les équations (3) et (4) permettent de comparer l'énergie maximale convertie par cycle avec le circuit de la FIGURE 1 et celle convertie avec le circuit selon l'invention dans la version circuit de base de la FIGURE 4. Ainsi qu'illustré en FIGURE 15, ce circuit de base selon l'invention peut présenter des performances proches du circuit de la FIGURE 1 (Yen), voire légèrement inférieures.

Cependant, le circuit selon l'invention présente plusieurs avantages par rapport au circuit de la FIGURE 1, même en version de base de la FIGURE 4, car il n'utilise ni interrupteur, ni inductance. Toute l'énergie générée est fournie directement à l'élément de stockage, sans condensateur de stockage intermédiaire ni circuit de retour. On obtient ainsi de meilleures possibilités de miniaturisation, une plus grande simplicité, potentiellement une meilleure fiabilité. Cette simplicité peut en outre aussi apporter de meilleures performances en conditions réelles.

Variantes du circuit de base

**Circuit de base "inversé"**

En FIGURE 7 est illustré le circuit d'alimentation d'un dispositif formant une version inversée du circuit de base de la FIGURE 4.

Dans ce dispositif, le circuit d'alimentation comprend les éléments suivants, et de préférence uniquement ceux-ci :

- une branche de génération 21 comprenant le condensateur variable Cv et un ou plusieurs condensateur de polarisation C1, et de préférence uniquement ceux-ci (et de préférence en un seul exemplaire), ladite branche de génération étant reliée à la masse par le noeud de base NB ;
- un circuit de redressement 23 comprenant les composants suivants, et de préférence uniquement ceux-ci :

   o une diode D2' (unique de préférence), ou plusieurs diodes montées dans le même sens (de préférence en série), vers le noeud de base NB et depuis le noeud de sortie S de l'extrémité de la branche de génération 21 située du côté du condensateur de polarisation C1,
   o une diode D1' (unique de préférence), ou plusieurs diodes montées dans le même sens (de préférence en série), vers le noeud de sortie S de l'extrémité de la branche de génération 21 située du côté du condensateur de polarisation et depuis l'extrémité OUT de la branche de stockage 24 située du côté opposé au noeud de base NB ;

- une branche de stockage 24 comprenant un élément de stockage, et de préférence uniquement celui-ci, ledit élément de stockage comprenant un condensateur C2 ou un élément de stockage chimique (et de préférence uniquement l'un d'eux) ; et
- une branche de retour de charge 22 comprenant une diode D3' (unique de préférence), ou plusieurs diodes montées dans le même sens (de préférence en série), vers l'extrémité OUT de la branche de stockage 24 située du côté opposé au noeud de base NB et depuis le noeud de polarisation (P) situé dans la branche de génération 21 entre le condensateur variable Cv et le condensateur de polarisation C1.

Ce circuit est une transposition directe du circuit de base de la FIGURE 4, obtenue en inversant toutes les diodes. Son fonctionnement et ses caractéristiques sont identiques au précédent, mais les polarités de toutes les tensions sont inversées, ce qui peut présenter certains avantages en fonction du contexte technologique, par exemple l'architecture et les autres composants du dispositif dans lequel il est intégré.

**Circuit de base avec stockage "à point milieu"**

En FIGURE 8 est illustré le circuit d'alimentation d'un dispositif réalisé à partir du circuit de base et dans lequel la capacité de stockage est d'un type à point milieu branché sur le redresseur.

Le circuit d'alimentation de ce dispositif comprend un composant de stockage d'énergie électrique (de préférence formé par deux condensateurs connectés en série) présentant un point intermédiaire en tension, formant par exemple un point milieu, qui est branché au noeud de base NB et délimite ainsi :

- une première partie de stockage C2 qui forme l'élément de stockage de la branche de stockage 24 ; et
- une deuxième partie de stockage C3 qui est monté en série dans le circuit de redressement 23 entre le noeud de base NB et le noeud de polarisation S situé dans la branche de génération 21 entre le condensateur variable Cv et le condensateur de polarisation C1.

Dans cette configuration, la tension Vmax est égale à 3/2*Vout et la tension Vmin est égale à 1/2*Vout.

L'énergie convertie par cycle s'exprime donc de la manière suivante :

$$W\max = \frac{Vout^2}{2}.C\min\left(\frac{C\max}{C\min} - 3\right) \tag{5}$$

L'énergie générée par cycle est donc inférieure à celle du circuit de base de la FIGURE 4, mais cette architecture peut présenter d'autres avantages, par exemple la disponibilité de plusieurs tensions en sortie du dispositif de stockage ou la répartition spatiale des différentes parties de stockage.

**Circuit de base avec interrupteur**

En FIGURE 9 est illustré le circuit d'alimentation d'un dispositif comprenant le circuit de base de la FIGURE 4 muni en outre d'un interrupteur.

Dans le circuit d'alimentation de ce dispositif, le circuit de redressement 24 comprend, dans une branche formant un sens unique entre le noeud de base NB et le noeud de sortie S de l'extrémité de la branche de génération 21 située du côté du condensateur de polarisation C1, un composant K formant interrupteur qui est agencé pour pouvoir ouvrir ladite branche à la demande d'un utilisateur ou de façon commandée selon une période de temps comprenant une pluralité de cycles du condensateur variable Cv, et par exemple plus de 100 voire 1000 cycles, mais pas forcément un nombre entier de cycles.

Ainsi, le circuit d'alimentation présente une "durée passive" (comme définie précédemment) qui correspond la période d'actionnement de cet interrupteur. Cet interrupteur est par exemple commandé par un circuit de temporisation indépendant du fonctionnement du condensateur variable, par exemple un simple circuit RC. Dans ce cas, le fonctionnement du circuit d'alimentation peut aussi être considéré comme autosynchrone même sur une durée couvrant l'actionnement de l'interrupteur, et par exemple de façon permanente.

Ce circuit est déduit du circuit de base de la FIGURE 4, en ajoutant un interrupteur K placé en série avec la diode D2. Lorsque l'interrupteur K est ouvert, le dispositif ne génère pas d'énergie mais il précharge l'élément de polarisation C1 à une tension qui tend vers :

$$Vout.\left(\frac{C\max}{C\min} - 1\right)$$

Lorsque l'interrupteur est fermé, le dispositif génère de l'énergie de la même façon que le circuit de base.

Le fait d'ouvrir périodiquement l'interrupteur K permet de compenser les diverses imperfections du circuit, de manière à maintenir une tension de polarisation aussi élevée que possible. Cela peut est programmé par exemple toutes les minutes ou toutes les heures, automatiquement ou manuellement ou lors d'un mouvement particulier ou d'une absence de mouvement, par exemple pour le cas d'un stimulateur cardiaque.

Dans la configuration où l'interrupteur K est fermé et où l'élément de polarisation C1 est chargé à son maximum, c'est à dire avec une tension égale à $Vout.\left(\frac{C\max}{C\min} - 1\right)$, la tension Vmax est égale à Vout*Cmax/Cmin et la tension Vmin est égale à $Vout.\left(\frac{C\max}{C\min} - 1\right)$.

L'énergie convertie par cycle s'exprime donc de la manière suivante :

$$W\max = Vout^2.C\max\left(\frac{C\max}{C\min} - 2\right) \tag{6}$$

Sur les courbes représentées sur la FIGURE 15, on voit que ce circuit produit plus d'énergie par cycle que le circuit de la FIGURE 1 lorsque la valeur du rapport Cmax/Cmin est comprise entre 1 et 5.

# EP 3 170 252 B1

Modes de réalisation avec multiplicateur de tension

L'invention propose en outre d'intégrer un circuit de type multiplicateur de tension au sein du circuit d'alimentation électrique du dispositif. Cette caractéristique d'utilisation d'un multiplicateur de tension est ici proposée avec l'architecture du circuit de base de la FIGURE 4. L'invention prévoit aussi de l'utiliser en combinaison avec les différentes variantes décrites pour ce circuit de base, en particulier avec les deuxième, troisième et quatrième modes de réalisation.

En FIGURE 10a est illustré le circuit d'alimentation d'un dispositif selon l'invention, dans un exemple particulier d'un mode de réalisation préféré de l'invention. Cet exemple de mode de réalisation comprend lui-même toute une famille de circuits comprenant un multiplicateur de tension, lequel peut être constitué et branché de différentes façons.

Dans cette famille à multiplicateur, le circuit d'alimentation électrique 12m comprend un circuit MT agencé pour former un circuit multiplicateur de tension, qui est connecté à la branche de génération 21 par le noeud de polarisation P situé entre le condensateur variable 211 et le condensateur de polarisation 212. Ce multiplicateur MT est agencé et branché pour appliquer à ce noeud de polarisation P une tension multipliée par rapport à la tension existant entre

- d'une part le noeud de sortie S de la branche de génération 21, et
- d'autre part : soit le noeud de base NB soit l'une des extrémités de la branche de stockage 24, par exemple l'extrémité OUT située entre la branche de stockage 24 et le redresseur 23 du côté du condensateur de polarisation 212.

Cette famille à multiplicateurs peut être mise en oeuvre avec les différents types de circuits multiplicateurs MT, en particulier ceux à trois ports, et dans de nombreuses configurations dont certaines sont détaillées plus loin. Ces différents multiplicateurs et leurs branchements sont applicables à de nombreuses variantes du circuit d'alimentation décrit en FIGURE 3, en particulier mais non exhaustivement le circuit "de base" de la FIGURE 4 ainsi que ceux décrits aux FIGURE 7, FIGURE 8 et FIGURE 9.

Avec certains types de multiplicateurs et selon certains branchements, le rôle de la branche de retour de charge 22 est rempli par le multiplicateur MT et ne comprend pas de composants ou conducteurs qui lui soient propres, comme par exemple en FIGURE 10b décrite ci-dessous.

La FIGURE 10b illustre ainsi un exemple de mise en oeuvre à partir du circuit de la FIGURE 4 appelé ici "circuit de base", avec un multiplicateur simple d'un type dit parallèle à deux cellules en cascade. Dans cet exemple, le circuit de charge 22 de l'élément de polarisation C1 est un multiplicateur de tension à base de diodes (de préférence des diodes D toutes identiques entre elles et avec les autres diodes du circuit d'alimentation) et de condensateurs. La tension Vout est une tension continue. La tension Ve est une tension qui varie périodiquement entre 0 et Vout. Chaque cellule ajoute une tension Vout. Ainsi, au noeud de polarisation P, la tension de polarisation Vc1 est égale à $(n+1)*Vout$, avec 'n' étant le nombre de cellules. Le nombre de cellules peut être choisi suivant les besoins. La tension Vmax est égale à $(n+1)*Vout$ et la tension Vmin est égale à $n*Vout$.

Cet exemple figure le cas d'un multiplicateur de tension à deux cellules MC1 et MC2 (n=2), ce qui donne une tension de polarisation $V_{c1}=3.V_{OUT}$. De ce fait, l'énergie convertie par cycle s'exprime de la manière suivante :

$$W = n.Vout^2.C\min\left(\frac{C\max}{C\min} - \frac{n+1}{n}\right) \qquad (7)$$

**Evaluation des performances**

En FIGURE 15 sont représentées les performances théoriques d'énergie par cycle, obtenues par simulation pour différents modes de réalisation du circuit d'alimentation électrique d'un dispositif selon l'invention.

On voit que les modes de réalisation à multiplicateurs peuvent améliorer les performances théoriques par rapport au circuit de base, mais il est à noter que c'est au prix d'une plus grande complexité, pouvant être cause d'encombrement et aussi de plus grandes pertes parasites. Ces solutions peuvent être préférées par exemple dans le cas d'un plus grand espace disponible, ou par exemple si la capacité variable présente un faible rapport Cmax/Cmin (par exemple inférieur à 2). Les versions de base ou avec peu de cellules peuvent être préférées par exemple pour une plus grande miniaturisation, ou si la capacité variable présente un grand rapport Cmax/Cmin (par exemple supérieur à 2).

**Différents types de multiplicateurs**

Plus généralement, l'invention propose toute une famille de circuits d'alimentation, répartie en quatre modes de réalisation couvrant chacun un type de branchement du multiplicateur de tension. Chacun de ces modes de réalisation de l'invention peut être mise en oeuvre avec des multiplicateurs avec plus ou moins de cellules, produisant différents facteurs de multiplication.

Les FIGURE 11 à FIGURE 14 illustrent ainsi ces quatre modes de réalisation de l'invention dans lesquels le circuit d'alimentation comprend un circuit agencé pour former un circuit multiplicateur de tension qui est connecté à la branche de génération entre le condensateur variable et le condensateur de polarisation pour y appliquer une tension multipliée par rapport à la tension existant entre :

- d'une part le noeud de sortie S de la branche de génération 21, et

- d'autre part le noeud de base ou l'une des extrémités de la branche de stockage.

Un tel multiplicateur de tension peut être de différents types, par exemples des types connus. En particulier, il est prévu que le circuit multiplicateur de tension soit un multiplicateur en cascade d'un type série ou en cascade d'un type parallèle.

**Multiplicateur intégré selon l'architecture de type 1**

En FIGURE 11 est illustré le circuit d'alimentation d'un dispositif selon l'invention, dans un cinquième mode de réalisation réalisé à partir du circuit de base de la FIGURE 4, dans une version selon une variante appelée type 1. Dans ce mode de réalisation, le circuit d'alimentation comprend un circuit multiplicateur de tension MT1 intégré dans la branche de retour de charge 22 depuis le dispositif de stockage 24 vers le noeud de polarisation P situé dans la branche de génération 21 entre la capacité variable Cv et le condensateur de polarisation C1.
Dans ce mode de réalisation, le circuit multiplicateur comprend un multiplicateur en cascade qui contribue à la fonction de circuit de retour de charge, par exemple monté en série avec le ou au sein du circuit de retour de charge 22. Ce circuit multiplicateur MT1 comprend au moins un premier noeud de branchement M1, un deuxième noeud de branchement M2 et un troisième noeud de branchement M3. De la même façon que dans l'exemple de la FIGURE 10b, il est monté selon une première configuration de façon à ce que :

- un premier M1 et un deuxième M2 noeuds de branchement forment entre eux une branche apte à conduire un courant unidirectionnel (c'est-à-dire toujours dans le même sens, même s'il est interrompu dans le temps), représenté par la flèche en trait gras. Ces deux noeuds sont connectés

    o pour l'un M2, au noeud de polarisation P situé dans la branche de génération 21 entre le condensateur variable Cv et le condensateur de polarisation C1, et
    o pour l'autre M1, à l'extrémité OUT de la branche de stockage 24 du côté opposé au noeud de base NB ;

- un troisième noeud est connecté au noeud de sortie S.

**Multiplicateur intégré selon l'architecture de type 2**

En FIGURE 12 est illustré le circuit d'alimentation d'un dispositif selon l'invention, dans un sixième mode de réalisation réalisé à partir du circuit de base de la FIGURE 4, dans une version selon une variante appelée type 2. Dans ce mode de réalisation, le circuit d'alimentation comprend un circuit multiplicateur de tension MT2 intégré dans la branche de retour de charge 22 depuis le noeud de sortie S vers la capacité variable Cv.
Dans ce mode de réalisation, le circuit multiplicateur MT2 comprend un multiplicateur en cascade qui contribue au circuit de retour de charge et qui comprend au moins un premier, un deuxième et un troisième noeuds de branchement M1, M2, M3 et est monté selon une deuxième configuration de façon à ce que :

- le premier M1 et le deuxième M2 noeuds de branchement forment entre eux une branche apte à conduire un courant unidirectionnel par exemple un courant continu, représenté par la flèche en trait gras. Ces deux noeuds sont connectés

    ◦ pour l'un M1 au noeud de polarisation P situé dans la branche de génération 21 entre le condensateur variable Cv et le condensateur de polarisation C1, et
    ◦ pour l'autre M2 au noeud de sortie S ;

- le troisième noeud de branchement M3 est connecté à l'extrémité OUT de la branche de stockage 24 du côté opposé au noeud de base NB.

**Multiplicateur intégré selon l'architecture de type 3**

En FIGURE 13 est illustré le circuit d'alimentation d'un dispositif selon l'invention, dans un septième mode de réalisation réalisé à partir du circuit de base de la FIGURE 4, dans une version selon une variante appelée type 3. Dans ce mode de réalisation, le circuit d'alimentation comprend un circuit multiplicateur de tension MT3 intégré dans la branche de génération 21 depuis le noeud de sortie S vers la capacité variable Cv.

Dans ce mode de réalisation, le circuit multiplicateur MT3 est un multiplicateur en cascade qui comprend au moins un premier, un deuxième et un troisième noeuds de branchement M1, M2, M3 et est monté selon une troisième configuration de façon à ce que :

- le premier M1 et le deuxième M2 noeuds de branchement forment entre eux une branche apte à conduire un courant unidirectionnel, représenté par la flèche en trait gras. Ces deux noeuds sont connectés

  ○ pour l'un M1 au noeud de polarisation P situé dans la branche de génération 21 entre le condensateur variable Cv et le condensateur de polarisation C1, et
  ○ pour l'autre M2 au noeud de sortie S ;

- le troisième noeud de branchement M3 est connecté au noeud de base NB.

**Multiplicateur intégré selon l'architecture de type 4**

En FIGURE 14 est illustré le circuit d'alimentation d'un dispositif selon l'invention, dans un huitième mode de réalisation réalisé à partir du circuit de base de la FIGURE 4, dans une version selon une variante appelée type 4. Dans ce mode de réalisation, le circuit d'alimentation comprend un circuit multiplicateur de tension MT4 intégré dans la branche de génération 21 depuis le noeud de base NB vers la capacité variable CV.

Dans ce mode de réalisation, le circuit multiplicateur MT4 est un multiplicateur en cascade qui comprend au moins un premier, un deuxième et un troisième noeuds de branchement M1, M2, M3 et est monté selon une quatrième configuration de façon à ce que :

- le premier M1 et le deuxième M2 noeuds de branchement forment entre eux une branche apte à conduire un courant unidirectionnel, représenté par la flèche en trait gras. Ces deux noeuds sont connectés

  ○ pour l'un M1 au noeud de polarisation P situé dans la branche de génération 21 entre le condensateur variable Cv et le condensateur de polarisation C1, et
  ○ pour l'autre M2 au noeud de base NB ;

- le troisième noeud de branchement M3 est connecté au noeud de sortie S.

**Exemples de circuits avec multiplicateurs**

Les FIGURE 16 à FIGURE 32 représentant des exemples particuliers de circuit d'alimentation proposés pour un dispositif selon l'invention, dans l'un des modes de réalisation avec multiplicateur des types 1 à 4, avec un élément de stockage V3 pouvant être aussi un accumulateur électrochimique.

En FIGURE 16 à FIGURE 17 : avec un multiplicateur de tension de d'un facteur 1, c'est-à-dire avec Vc1=Vout. Ce type de multiplicateur est proposé en version de type 1 en FIGURE 16 et de type 2 en FIGURE 17. Il peut être considéré comme un type parallèle ou un type série.

En FIGURE 18 à FIGURE 21 : avec un multiplicateur de tension de type parallèle d'un facteur 2, c'est-à-dire avec Vc1=2.Vout. Ce type de multiplicateur est proposé dans les versions suivantes :

- en FIGURE 18, de type 1,
- en FIGURE 19, de type 2,
- en FIGURE 20, de type 3,
- en FIGURE 21, de type 4,

En FIGURE 22 et FIGURE 23 : avec un multiplicateur de tension de type parallèle d'un facteur 3, c'est-à-dire avec Vc1=3.Vout. Ce type de multiplicateur est proposé en version de type 1 en FIGURE 22 (dont le circuit est identique à celui de la FIGURE 10b), et de type 2 en FIGURE 23.

En FIGURE 24 et FIGURE 25 : avec un multiplicateur de tension de type parallèle d'un facteur 4, c'est-à-dire avec Vc1=4.Vout. Ce type de multiplicateur est proposé en version de type 1 en FIGURE 24 et de type 2 en FIGURE 25.

En FIGURE 26 à FIGURE 29 : avec un multiplicateur de tension de type série d'un facteur 2, c'est-à-dire avec Vc1=2.Vout. Ce type de multiplicateur est proposé dans les versions suivantes :

- en FIGURE 26, de type 1,
- en FIGURE 27, de type 2,
- en FIGURE 28, de type 3,
- en FIGURE 29, de type 4.

En FIGURE 30 et FIGURE 31 : avec un multiplicateur de tension de type série d'un facteur 3, c'est-à-dire avec Vc1=3.Vout. Ce type de multiplicateur est proposé en version de type 1 en FIGURE 30 et de type 2 en FIGURE 31.

En FIGURE 24 et FIGURE 25 : avec un multiplicateur de tension de type parallèle d'un facteur 4, c'est-à-dire avec Vc1=4.Vout. Ce type de multiplicateur est proposé en version de type 1 en FIGURE 24 et de type 2 en FIGURE 25.

Variantes avec redresseur en pont complet

En FIGURE 32 et FIGURE 33 sont représentés les circuits d'alimentation d'un dispositif selon l'invention de l'un des modes de réalisation avec multiplicateur, dans des versions avec un circuit redresseur formé par un pont "complet" à 4 diodes, et :

- en FIGURE 32, un multiplicateur de tension de type 3 de type parallèle et d'un facteur 4 ;
- en FIGURE 33, un multiplicateur de tension de type 4 de type série et d'un facteur 3.

Dans ces modes de réalisation, le circuit de redressement (23') comprend au moins un pont d'au moins quatre diodes formant :

- au moins deux branches (D1 et D7, ou D1 et D9) formant une connexion unidirectionnelle vers l'extrémité (OUT) de la branche de stockage (24) du côté de la branche de retour de charge (22), depuis le point de sortie (S) et respectivement depuis le point de base (NB) ; et
- au moins deux branches (D2 et D8, ou D2 et D10) formant une connexion unidirectionnelle depuis l'extrémité de la branche de stockage (24) du côté opposé à la branche de retour de charge (22), vers le point de sortie (S) et respectivement vers le point de base (NB).

**Exemples d'applications**

A titre d'ordre de grandeur, un tel circuit d'alimentation permet d'envisager un rendement énergétique de plus de 80% pour une tension de stockage de 5V. Le dispositif selon l'invention est envisagé plus particulièrement pour réaliser, ou être intégré dans, par exemple :

- un implant médical, tel que stimulateur cardiaque ou appareil surveillance implanté ;
- un appareil embarqué dans un élément portable, tel que vêtement, chaussure, bijou, bracelet, bandeau, oreillette ;
- un appareil transmetteur d'information par détection, mesure ou surveillance d'environnement, tel qu'un capteur fixe ou dispersé, ou un répéteur d'information ou capteur autonome embarqué dans un véhicule.

Bien sûr, l'invention n'est pas limitée aux exemples qui viennent d'être décrits et de nombreux aménagements peuvent être apportés à ces exemples sans sortir du cadre de l'invention, telle que définie par les revendications.

**Revendications**

1. Dispositif électronique (1) de type fonctionnant sans apport d'énergie électrique extérieure, comprenant un circuit d'alimentation électrique (12m) agencé pour alimenter ledit dispositif, ledit circuit d'alimentation électrique étant agencé pour alimenter en énergie le fonctionnement dudit dispositif électronique à travers un élément de stockage (13, 24, C2, V3),

   ledit circuit d'alimentation électrique (12m) comprenant au moins un composant, dit condensateur variable (11, 211, Cv, Cvar), présentant une capacité électrique variable sous l'effet d'un mouvement mécanique alternatif (10), ledit condensateur variable

o étant agencé pour recevoir ledit mouvement mécanique (10) de la part de l'environnement dudit dispositif électronique (1),
o et étant connecté audit dispositif de stockage (13, 24, C2, V3) au moyen d'un circuit de redressement (23, 23') pour augmenter l'énergie électrique qui y est stockée ;

dans lequel ledit circuit d'alimentation électrique (12m) comprend :

- une branche de génération (21) formée par au moins le condensateur variable (211, Cv) et un condensateur de polarisation (212, C1) montés en série, laquelle branche de génération est montée en parallèle avec le circuit de redressement (23, 23') et avec une branche de stockage (24) comprenant l'élément de stockage, entre

  ◦ un noeud dit de base (NB) qui est situé à l'extrémité de la branche de génération (21) du côté du condensateur variable (211, Cv) et est maintenu à un potentiel de référence,
  ◦ un noeud dit de sortie (S) situé à l'extrémité de la branche de génération (21) du côté du condensateur de polarisation (211, C1) ;

- une branche de retour de charge (22) d'un type apte à conduire un courant électrique unidirectionnel :

  ◦ vers la branche de génération (21), par au moins une première extrémité connectée à un noeud (P) dit de polarisation, qui est situé dans la branche de génération entre le condensateur variable (211, Cv, Cvar) et le condensateur de polarisation (212, C1) ;
  ◦ depuis le redresseur (23), par au moins une deuxième extrémité recevant une partie de l'énergie électrique produite par ledit circuit d'alimentation (12m),

dans lequel l'ensemble formé par ladite branche de génération (21), le circuit de redressement (23, 23'), la branche de stockage (24) et la branche de retour de charge (22) constitue un circuit électrique dont le comportement est passif et demeure inchangé et permanent dans ses caractéristiques pendant au moins une pluralité de cycles du condensateur variable (211, Cv, Cvar),
réalisant ainsi à chaque cycle un complément de charge du condensateur de polarisation (212, C1) selon un mode de fonctionnement qui est autosynchrone, c'est-à-dire qu'il ne nécessite aucun moyen de synchronisation par rapport aux mouvements mécaniques pendant ladite pluralité de cycles ;
et étant **caractérisé en ce que** le circuit d'alimentation électrique (12m) comprend un circuit agencé pour former un circuit multiplicateur de tension (MT, MT1, MT2, MT3, MT4) qui est connecté à la branche de génération (21) en un noeud de polarisation (P) situé entre le condensateur variable (211, Cv, Cvar) et le condensateur de polarisation (212, C1), pour y appliquer une tension multipliée par rapport à la tension existant entre

- d'une part le noeud de sortie (S) de ladite branche de génération (21), et
- d'autre part le noeud de base (NB) ou l'une (OUT) des extrémités de la branche de stockage (24, C2, V3).

2. Dispositif selon la revendication précédente, **caractérisé en ce que** la branche de retour de charge (22) comprend une diode orientée (D3, D3') depuis le redresseur (23, 23') vers la branche de génération (21), ou plusieurs diodes (D, D3 à D9 FIGURE 16 à FIGURE 31) montées dans le même sens, et de préférence en série.

3. Dispositif selon l'une quelconque des revendications précédentes, **caractérisé en ce que** le circuit d'alimentation électrique (12m), ou au moins un ou plusieurs des circuits parmi la branche de génération (21), la branche de stockage (24), la branche de retour de charge (22) et le circuit de redressement (23, 23'), est réalisé de façon à ne comprendre que des composants passifs et des semi-conducteurs non commandés, et en particulier uniquement des diodes et des condensateurs.

4. Dispositif selon l'une quelconque des revendications précédentes, **caractérisé en ce que** le circuit d'alimentation (12m) comprend les éléments suivants, et de préférence uniquement ceux-ci :

- une branche de génération (21) comprenant le condensateur variable (211, Cv, Cvar) et un ou plusieurs condensateurs (212, C1) de polarisation, et de préférence uniquement ceux-ci, ladite branche de génération (21) étant reliée par son noeud de base (NB) à un potentiel de référence, par un noeud qui est commun au redresseur (23) et à la branche de stockage (24) ;
- un circuit de redressement (23) comprenant les composants suivants, et de préférence uniquement ceux-ci :

∘ une diode (D2), ou plusieurs diodes montées dans le même sens, depuis le noeud de base (NB) et vers l'extrémité (S) de la branche de génération (21) située du côté du condensateur de polarisation (212, C1),
∘ une diode (D1), ou plusieurs diodes montées dans le même sens, depuis l'extrémité (S) de la branche de génération (21) située du côté du condensateur de polarisation (212, C1) et vers l'extrémité (OUT) de la branche de stockage (24) située du côté opposé au noeud de base (NB) ;

- une branche de stockage (24) comprenant un élément de stockage, et de préférence uniquement celui-ci, ledit élément de stockage comprenant un condensateur (C2) ou un élément de stockage chimique ; et
- une branche de retour de charge (22) comprenant une diode (D3, D3'), ou plusieurs diodes (D, D3 à D9 FIGURE 16 à FIGURE 31) montées dans le même sens, depuis l'extrémité (OUT) de la branche de stockage située du côté opposé au noeud de base (NB) et vers un noeud de polarisation (P) de la branche de génération (21) situé entre le condensateur variable (211, Cv) et le condensateur de polarisation (212, C1).

5. Dispositif selon l'une quelconque des revendications 1 à 3, **caractérisé en ce que** le circuit d'alimentation (12m) comprend les éléments suivants, et de préférence uniquement ceux-ci :

- une branche de génération (21) comprenant le condensateur variable (211, Cv) et un ou plusieurs condensateur de polarisation (C1), et de préférence uniquement ceux-ci, ladite branche de génération étant reliée à la masse ou à un potentiel de référence par le noeud de base (NB) ;
- un circuit de redressement (23) comprenant les composants suivants, et de préférence uniquement ceux-ci :

∘ une diode (D2'), ou plusieurs diodes montées dans le même sens, vers le noeud de base (NB) et depuis l'extrémité (S) de la branche de génération située du côté du condensateur de polarisation,
∘ une diode (D1'), ou plusieurs diodes montées dans le même sens, vers l'extrémité (S) de la branche de génération située du côté du condensateur de polarisation et depuis l'extrémité (OUT) de la branche de stockage (24) située du côté opposé au noeud de base (NB) ;

- une branche de stockage (24) comprenant un élément de stockage, et de préférence uniquement celui-ci, ledit élément de stockage comprenant un condensateur (C2) ou un élément de stockage chimique ; et
- une branche de retour de charge (22) comprenant une diode (D3'), ou plusieurs diodes montées dans le même sens, vers l'extrémité (OUT) de la branche de stockage située du côté opposé au noeud de base et depuis un noeud de polarisation (P) de la branche de génération (21) situé entre le condensateur variable (211, Cv) et le condensateur de polarisation (212, C1).

6. Dispositif selon la revendication 1, **caractérisé en ce que** le circuit d'alimentation (12m) comprend un composant de stockage d'énergie électrique présentant un point intermédiaire en tension, formant par exemple un point milieu, qui est branché au noeud de base (NB) et délimite ainsi :

- une première partie de stockage (C2) qui forme l'élément de stockage de la branche de stockage ; et
- une deuxième partie de stockage (C3) qui est monté en série dans le circuit de redressement (23) entre le noeud de base (NB) et un noeud de polarisation (P) de la branche de génération (21) situé entre le condensateur variable (211, Cv) et le condensateur de polarisation (212, C1).

7. Dispositif selon l'une quelconque des revendications précédentes, **caractérisé en ce que** le circuit de redressement (23) comprend, dans une branche formant un sens unique entre le noeud de base (NB) et l'extrémité (S) de la branche de génération (21) située du côté du condensateur de polarisation (212, C1), un composant (K) formant interrupteur et qui est agencé pour pouvoir ouvrir ladite branche à la demande d'un utilisateur ou de façon commandée selon une période de temps comprenant une pluralité de cycles du condensateur variable, et notamment plus de 100 voire 1000 cycles.

8. Dispositif selon l'une quelconque des revendications précédentes, **caractérisé en ce que** le circuit multiplicateur de tension est un multiplicateur en cascade d'un type série (MT1, MT2, MT3) ou parallèle (MT1, MT2, MT3, MT4).

9. Dispositif selon la revendication précédente, **caractérisé en ce que** le circuit multiplicateur (MT1) comprend un multiplicateur en cascade qui contribue à la fonction de circuit de retour de charge (22) et qui comprend au moins un premier, un deuxième et un troisième noeuds de branchement (M1, M2, M3) et est monté selon une première configuration de façon à ce que :

- le premier (M1) et le deuxième (M2) noeuds de branchement forment entre eux une branche apte à conduire un courant unidirectionnel, et sont connectés

    ◦ pour l'un (M1), à un noeud de polarisation (P) situé dans la branche de génération (21) entre le condensateur variable (211, Cv, Cvar) et le condensateur de polarisation (212, C1), et
    ◦ pour l'autre (M2), à l'extrémité (OUT) de la branche de stockage (24, C2, V3) du côté opposé au noeud de base (NB) ;

- le troisième noeud de branchement (M3) est connecté au noeud de sortie (S).

**10.** Dispositif selon l'une quelconque des revendications 1 à 8, **caractérisé en ce que** le circuit multiplicateur (MT2) comprend un multiplicateur en cascade qui contribue au circuit de retour de charge et qui comprend au moins un premier, un deuxième et un troisième noeuds de branchement (M1, M2, M3) et est monté selon une deuxième configuration de façon à ce que :

- le premier (M1) et le deuxième (M2) noeuds de branchement forment entre eux une branche apte à conduire un courant unidirectionnel, et sont connectés

    ◦ pour l'un (M1), à un noeud de polarisation (P) situé dans la branche de génération (21) entre le condensateur variable (211, Cv, Cvar) et le condensateur de polarisation (212, C1), et
    ◦ pour l'autre (M2), au noeud de sortie (S) ;

- le troisième noeud de branchement (M3) est connecté à l'extrémité (OUT) de la branche de stockage (24, V4) du côté opposé au noeud de base (NB).

**11.** Dispositif selon l'une quelconque des revendications 1 à 8, **caractérisé en ce que** le circuit multiplicateur (MT3) est un multiplicateur en cascade qui comprend au moins un premier, un deuxième et un troisième noeuds de branchement (M1, M2, M3) et est monté selon une troisième configuration de façon à ce que :

- le premier (M1) et le deuxième (M2) noeuds de branchement forment entre eux une branche apte à conduire un courant unidirectionnel, et sont connectés

    ◦ pour l'un (M1), à un noeud de polarisation (P) situé dans la branche de génération (21) entre le condensateur variable (211, Cv, Cvar) et le condensateur de polarisation (212, C1), et
    ◦ pour l'autre (M2), au noeud de sortie (S) ;

- le troisième noeud de branchement (M3) est connecté au noeud de base (NB).

**12.** Dispositif selon l'une quelconque des revendications 1 à 8, **caractérisé en ce que** le circuit multiplicateur (MT4) est un multiplicateur en cascade qui comprend au moins un premier, un deuxième et un troisième noeuds de branchement (M1, M2, M3) et est monté selon une quatrième configuration de façon à ce que :

- le premier (M1) et le deuxième (M2) noeuds de branchement forment entre eux une branche apte à conduire un courant unidirectionnel, et sont connectés

    ◦ pour l'un (M1), à un noeud de polarisation P situé dans la branche de génération (21) entre le condensateur variable (211, Cv, Cvar) et le condensateur de polarisation (212, C1), et
    ◦ pour l'autre (M2), au noeud de base (NB) ;

- le troisième noeud de branchement (M3) est connecté au noeud de sortie (S).

**13.** Dispositif selon l'une quelconque des revendications précédentes, **caractérisé en ce que** le condensateur de polarisation (212, C1) présente une capacité électrique qui vaut au moins cinq fois la variation de la capacité du condensateur variable (211, Cv, Cvar) au cours d'un cycle (10), et/ou **en ce que** l'élément de stockage présente une capacité de stockage d'énergie électrique correspondant à un condensateur (C2, V3) dont la capacité électrique est supérieure à trois fois celle du condensateur de polarisation (212, C1).

**14.** Dispositif selon l'une quelconque des revendications précédentes, **caractérisé en ce que** le circuit de redressement (23') comprend au moins un pont d'au moins quatre diodes formant :

- au moins deux branches (D1 et D7, ou D1 et D9) formant une connexion unidirectionnelle vers l'extrémité (OUT) de la branche de stockage (24) du côté de la branche de retour de charge (22), depuis le noeud de sortie (S) et respectivement depuis le point de base (NB) ; et
- au moins deux branches (D2 et D8, ou D2 et D10) formant une connexion unidirectionnelle depuis l'extrémité de la branche de stockage (24, V3) du côté opposé à la branche de retour de charge (22), vers le noeud de sortie (S) et respectivement vers le noeud de base (NB).

15. Circuit d'alimentation électrique (12m) agencé spécifiquement pour un dispositif électronique (1) selon l'une quelconque des revendications précédentes.

16. Dispositif électronique prévu pour être implanté dans le corps humain ou animal in vivo sans conserver de liaison physique avec l'extérieur, **caractérisé en ce qu'**il comprend ou forme un dispositif (1) selon l'une quelconque des revendications 1 à 14 ou comprend un circuit d'alimentation (12m) selon la revendication 15.

## Patentansprüche

1. Elektronische Vorrichtung (1), die ohne Außenzufuhr von elektrischer Energie betrieben wird, umfassend eine zur Versorgung der Vorrichtung angeordnete, elektrische Stromversorgungsschaltung (12m), wobei die elektrische Stromversorgungsschaltung dazu angeordnet ist, den Betrieb der elektronischen Vorrichtung durch ein Speicherelement (13, 24, C2, V3) mit Energie zu versorgen,
wobei die elektrische Stromversorgungsschaltung (12m) mindestens eine Komponente, einen sogenannten variablen Kondensator (11, 211, Cv, Cvar) umfasst, welcher eine unter der Wirkung einer mechanischen Hin- und Herbewegung (10) variable, elektrische Kapazität aufweist, wobei der variable Kondensator

   ◦ zum Empfang der mechanischen, von der Umgebung der elektronischen Vorrichtung (1) kommenden Bewegung (10) angeordnet ist,
   ◦ und mit der Speichervorrichtung (13, 24, C2, V3) mittels einer Gleichrichterschaltung (23, 23') zur Erhöhung der darin gespeicherten elektrischen Energie verbunden ist,

bei welcher die elektrische Stromversorgungsschaltung (12m) umfasst:

   - einen Erzeugungsabschnitt (21), der durch mindestens den variablen Kondensator (211, Cv) und einen Polarisierungskondensator (212, C1) gebildet ist, welche in Reihe geschaltet sind, wobei der Erzeugungsabschnitt mit der Gleichrichterschaltung (23, 23') und mit einem das Speicherelement umfassenden Speicherabschnitt (24) parallel geschaltet ist, und zwar zwischen

      ◦ einem sogenannten Basisknoten (NB), der am Ende des Erzeugungsabschnitts (21) auf der Seite des variablen Kondensators (211, Cv) gelagert ist und auf einem Referenzpotential gehalten wird,
      ◦ einem sogenannten Ausgangsknoten (S), der am Ende des Erzeugungsabschnitts (21) auf der Seite des Polarisierungskondensators (211, C1) gelagert ist,

   - einen Ladungsrückleitungsabschnitt (22), der dazu geeignet ist, einen einseitig gerichteten, elektrischen Strom wie folgt zu leiten:

      ◦ zum Erzeugungsabschnitt (21) hin über mindestens ein erstes, mit einem im Erzeugungsabschnitt zwischen dem variablen Kondensator (211, Cv, Cvar) und dem Polarisierungskondensator (212, C1) gelagerten, sogenannten Polarisierungsknoten (P) verbundene Ende,
      ◦ vom Gleichrichter (23) ausgehend über mindestens ein zweites Ende, das einen Teil der durch die Stromversorgungsschaltung (12m) erzeugten, elektrischen Energie empfängt,

bei welcher die durch den Erzeugungsabschnitt (21), die Gleichrichterschaltung (23, 23'), den Speicherabschnitt (24) und den Ladungsrückleitungsabschnitt (22) gebildete Anordnung eine elektrische Schaltung ausbildet, deren Verhalten passiv ist und in ihren Eigenschaften während mindestens einer Vielzahl von Zyklen des variablen Kondensators (211, Cv, Cvar) unverändert und gleich bleibt,
wobei sie somit bei jedem Zyklus den Polarisierungskondensator (212, C1) ergänzend auflädt, und zwar nach einem Betriebsmodus, der selbstsynchron ist, d.h. er braucht kein Synchronisierungsmittel gegenüber den mechanischen Bewegungen während der Vielzahl von Zyklen,
und **dadurch gekennzeichnet, dass** die elektrische Stromversorgungsschaltung (12m) eine Schaltung umfasst, welche zur Bildung einer Spannungsvervielfacherschaltung (MT, MT1, MT2, MT3, MT4) angeordnet ist, welche mit dem Erzeugungsabschnitt (21) an einem zwischen dem variablen Kondensator (211, Cv, Cvar) und dem Polarisierungskondensator (212, C1) gelagerten Polarisierungsknoten (P) verbunden ist, um dort eine Spannung anzulegen, die gegenüber der zwischen

- einerseits dem Ausgangsknoten (S) des Erzeugungsabschnitts (21) und
- andererseits dem Basisknoten (NB) oder einem (OUT) der Enden des Speicherabschnitts (24, C2, V3) bestehenden Spannung vervielfacht ist.

2. Vorrichtung nach dem vorhergehenden Anspruch, **dadurch gekennzeichnet, dass** der Ladungsrückleitungsabschnitt (22) umfasst: eine Diode (D3, D3'), welche vom Gleichrichter (23, 23') ausgehend zum Erzeugungsabschnitt (21) hin ausgerichtet ist, oder mehrere Dioden (D, D3 bis D9 ABBILDUNG 16 bis ABBILDUNG 31), die in der gleichen Richtung und vorzugsweise in Reihe geschaltet sind.

3. Vorrichtung nach einem der vorhergehenden Ansprüche, **dadurch gekennzeichnet, dass** die elektrische Stromversorgungsschaltung (12m) oder mindestens eine oder mehrere der Schaltungen unter der Gruppe aus dem Erzeugungsabschnitt (21), dem Speicherabschnitt (24), dem Ladungsrückleitungsabschnitt (22) und der Gleichrichterschaltung (23, 23') derart ausgeführt ist, dass sie ausschließlich passive Komponente und nicht gesteuerte Halbleiter und insbesondere ausschließlich Dioden und Kondensatoren umfasst.

4. Vorrichtung nach einem der vorhergehenden Ansprüche, **dadurch gekennzeichnet, dass** die Stromversorgungsschaltung (12m) folgende Elemente und vorzugsweise ausschließlich diese umfasst:

- einen Erzeugungsabschnitt (21) mit dem variablen Kondensator (211, Cv, Cvar) und einem oder mehreren Polarisierungskondensatoren (212, C1) und vorzugsweise nur mit diesen, wobei der Erzeugungsabschnitt (21) durch seinen Basisknoten (NB) mit einem Referenzpotential durch einen für den Gleichrichter (23) und den Speicherabschnitt (24) gemeinsamen Knoten verbunden ist,
- eine Gleichrichterschaltung (23) mit folgenden Komponenten und vorzugsweise ausschließlich diesen:

  ∘ einer Diode (D2) oder mehreren Dioden, welche vom Basisknoten (NB) ausgehend und zu dem auf der Seite des Polarisierungskondensators (212, C1) gelagerten Ende (S) des Erzeugungsabschnitts (21) hin in der gleichen Richtung geschaltet sind,
  ∘ einer Diode (D1) oder mehreren Dioden, welche vom auf der Seite des Polarisierungskondensators (212, C1) gelagerten Ende (S) des Erzeugungsabschnitts (21) ausgehend und zu dem auf der gegenüberliegenden Seite des Basisknotens (NB) gelagerten Ende (OUT) des Speicherabschnitts (24) hin in der gleichen Richtung geschaltet sind,

- einen Speicherabschnitt (24) mit einem Speicherelement und vorzugsweise ausschließlich mit diesem, wobei das Speicherelement einen Kondensator (C2) oder ein chemisches Speicherelement umfasst, und
- einen Ladungsrückleitungsabschnitt (22) mit einer Diode (D3, D3') oder mehreren Dioden (D, D3 bis D9 ABBILDUNG 16 bis ABBILDUNG 31), welche vom auf der gegenüberliegenden Seite des Basisknotens (NB) gelagerten Ende (OUT) des Speicherabschnitts ausgehend und zu einem zwischen dem variablen Kondensator (211, Cv) und dem Polarisierungskondensator (212, C1) gelagerten Polarisierungsknoten (P) des Erzeugungsabschnitts (21) hin in der gleichen Richtung geschaltet sind.

5. Vorrichtung nach einem der Ansprüche 1 bis 3, **dadurch gekennzeichnet, dass** die Stromversorgungsschaltung (12m) folgende Elemente und vorzugsweise ausschließlich diese umfasst:

- einen Erzeugungsabschnitt (21) mit dem variablen Kondensator (211, Cv) und einem oder mehreren Polarisierungskondensatoren (C1) und vorzugsweise ausschließlich mit diesen, wobei der Erzeugungsabschnitt geerdet oder über den Basisknoten (NB) mit dem Referenzpotenzial verbunden ist,
- eine Gleichrichterschaltung (23) mit folgenden Komponenten und vorzugsweise ausschließlich mit diesen:

  ∘ einer Diode (D2') oder mehreren Dioden, die zum Basisknoten (NB) hin und vom auf der Seite des Polarisierungskondensators gelagerten Ende (S) des Erzeugungsabschnitts ausgehend in der gleichen Richtung geschaltet sind,

◦ einer Diode (D1') oder mehreren Dioden, die zum auf der Seite des Polarisierungskondensators gelagerten Ende (S) des Erzeugungsabschnitts hin und vom auf der gegenüberliegenden Seite des Basisknotens (NB) gelagerten Ende (OUT) des Speicherabschnitts (24) ausgehend in der gleichen Richtung geschaltet sind,

- einen Speicherabschnitt (24) mit einem Speicherelement und vorzugsweise ausschließlich mit diesem, wobei das Speicherelement einen Kondensator (C2) oder ein chemisches Speicherelement umfasst, und
- einen Ladungsrückleitungsabschnitt (22) mit einer Diode (D3') oder mehreren Dioden, die zum auf der gegenüberliegenden Seite des Basisknotens gelagerten Ende (OUT) des Speicherabschnitts hin und von einem zwischen dem variablen Kondensator (211, Cv) und dem Polarisierungskondensator (212, C1) gelagerten Polarisierungsknoten (P) des Erzeugungsabschnitts (21) ausgehend in der gleichen Richtung geschaltet sind.

6. Vorrichtung nach Anspruch 1, **dadurch gekennzeichnet, dass** die Stromversorgungsschaltung (12m) eine Komponente zur Speicherung von elektrischer Energie umfasst, welches einen Spannungszwischenpunkt aufweist, welcher zum Beispiel einen Mittelabgriff bildet, der mit dem Basisknoten (NB) angeschlossen ist und somit folgendes begrenzt:

- einen ersten, das Speicherelement des Speicherabschnitts bildenden Speicherteil (C2), und
- einen zweiten Speicherteil (C3), der in der Gleichrichterschaltung (23) zwischen dem Basisknoten (NB) und einem zwischen dem variablen Kondensator (211, Cv) und dem Polarisierungskondensator (212, C1) gelagerten Polarisierungsknoten (P) des Erzeugungsabschnitts (21) in Reihe geschaltet ist.

7. Vorrichtung nach einem der vorhergehenden Ansprüche, **dadurch gekennzeichnet, dass** die Gleichrichterschaltung (23) in einem Abschnitt mit einseitig gerichteter Richtung zwischen dem Basisknoten (NB) und dem auf der Seite des Polarisierungskondensators (212, C1) gelagerten Ende (S) des Erzeugungsabschnitts (21) eine Komponente (K) als Schalter umfasst, welcher dazu angeordnet ist, auf Aufforderung eines Benutzers oder angesteuert gemäß einer eine Vielzahl von Zyklen des variablen Kondensators und insbesondere mehr als 100 bezüglich 1000 Zyklen umfassenden Zeitdauer den Abschnitt öffnen zu können.

8. Vorrichtung nach einem der vorhergehenden Ansprüche, **dadurch gekennzeichnet, dass** die Spannungsvervielfacherschaltung eine Hochspannungskaskade ist, welche in Reihe (MT1, MT2, MT3) oder parallel (MT1, MT2, MT3, MT4) geschaltet ist.

9. Vorrichtung nach dem vorhergehenden Anspruch, **dadurch gekennzeichnet, dass** die Spannungsvervielfacherschaltung (MT1) eine Hochspannungskaskade umfasst, welche zur Funktion als Ladungsrückleitungsschaltung (22) beiträgt und mindestens einen ersten, einen zweiten und einen dritten Anschlussknoten (M1, M2, M3) umfasst und nach einem ersten Aufbau derart geschaltet ist, dass

- der erste Anschlussknoten (M1) und der zweite Anschlussknoten (M2) untereinander einen Abschnitt bilden, welcher zur Leitung eines einseitig gerichteten Stroms geeignet ist, und wie folgt verbunden sind:

◦ der Eine (M1) mit einem Polarisierungsknoten (P), der in dem Erzeugungsabschnitt (21) zwischen dem variablen Kondensator (211, Cv, Cvar) und dem Polarisierungskondensator (212, C1) gelagert ist, und
◦ der Andere (M2) mit dem Ende (OUT) des Speicherabschnitts (24, C2, V3) auf der dem Basisknoten (NB) gegenüberliegenden Seite;

- der dritte Anschlussknoten (M3) mit dem Ausgangsknoten (S) verbunden ist.

10. Vorrichtung nach einem der Ansprüche 1 bis 8, **dadurch gekennzeichnet, dass** die Spannungsvervielfacherschaltung (MT2) eine Hochspannungskaskade umfasst, welche zur Ladungsrückleitungsschaltung beiträgt und mindestens einen ersten, einen zweiten und einen dritten Anschlussknoten (M1, M2, M3) umfasst und nach einem zweiten Aufbau derart geschaltet ist, dass

- der erste Anschlussknoten (M1) und der zweite Anschlussknoten (M2) untereinander einen Abschnitt bilden, welcher zur Leitung eines einseitig gerichteten Stroms geeignet ist, und wie folgt verbunden sind:

◦ der Eine (M1) mit einem Polarisierungsknoten (P), der in dem Erzeugungsabschnitt (21) zwischen dem variablen Kondensator (211, Cv, Cvar) und dem Polarisierungskondensator (212, C1) gelagert ist, und

∘ der Andere (M2) mit dem Ausgangsknoten (S);

- der dritte Anschlussknoten (M3) mit dem Ende (OUT) des Speicherabschnitts (24, V4) auf der dem Basisknoten (NB) gegenüberliegenden Seite verbunden ist.

**11.** Vorrichtung nach einem der Ansprüche 1 bis 8, **dadurch gekennzeichnet, dass** die Spannungsvervielfacherschaltung (MT3) eine Hochspannungskaskade ist, welche mindestens einen ersten, einen zweiten und einen dritten Anschlussknoten (M1, M2, M3) umfasst und nach einem dritten Aufbau derart geschaltet ist, dass

- der erste Anschlussknoten (M1) und der zweite Anschlussknoten (M2) untereinander einen Abschnitt bilden, welcher zur Leitung eines einseitig gerichteten Stroms geeignet ist, und wie folgt verbunden sind:

∘ der Eine (M1) mit einem Polarisierungsknoten (P), der in dem Erzeugungsabschnitt (21) zwischen dem variablen Kondensator (211, Cv, Cvar) und dem Polarisierungskondensator (212, C1) gelagert ist, und
∘ der Andere (M2) mit dem Ausgangsknoten (S);

- der dritte Anschlussknoten (M3) mit dem Basisknoten (NB) verbunden ist.

**12.** Vorrichtung nach einem der Ansprüche 1 bis 8, **dadurch gekennzeichnet, dass** die Spannungsvervielfacherschaltung (MT4) eine Hochspannungskaskade ist, welche mindestens einen ersten, einen zweiten und einen dritten Anschlussknoten (M1, M2, M3) umfasst und nach einem vierten Aufbau derart geschaltet ist, dass

- der erste Anschlussknoten (M1) und der zweite Anschlussknoten (M2) untereinander einen Abschnitt bilden, welcher zur Leitung eines einseitig gerichteten Stroms geeignet ist, und wie folgt verbunden sind:

∘ der Eine (M1) mit einem Polarisierungsknoten P, der in dem Erzeugungsabschnitt (21) zwischen dem variablen Kondensator (211, Cv, Cvar) und dem Polarisierungskondensator (212, C1) gelagert ist, und
∘ der Andere (M2) mit dem Basisknoten (NB);

- der dritte Anschlussknoten (M3) mit dem Ausgangsknoten (S) verbunden ist.

**13.** Vorrichtung nach einem der vorhergehenden Ansprüche, **dadurch gekennzeichnet, dass** der Polarisierungskondensator (212, C1) eine elektrische Kapazität aufweist, welche mindestens fünf Mal so hoch wie die Variation der Kapazität des variablen Kondensators (211, Cv, Cvar) während eines Zyklus (10), und/oder dadurch, dass das Speicherelement eine Speicherkapazität für die elektrische Energie entsprechend der eines Kondensators (C2, V3) aufweist, dessen elektrische Kapazität höher als drei Mal die des Polarisierungskondensators (212, C1) liegt.

**14.** Vorrichtung nach einem der vorhergehenden Ansprüche, **dadurch gekennzeichnet, dass** die Gleichrichterschaltung (23') mindestens eine Brücke mit mindestens vier Dioden umfasst, welche wie folgt bilden:

- mindestens zwei Abschnitte (D1 und D7, oder D1 und D9) als Einwegverbindung zum auf der Seite des Ladungsrückleitungsabschnitts (22) gelagerten Ende (OUT) des Speicherabschnitts (24) hin vom Ausgangsknoten (S) ausgehend und jeweils vom Basisknoten (NB) ausgehend; und
- mindestens zwei Abschnitte (D2 und D8, oder D2 und D10) als Einwegverbindung vom auf der dem Ladungsrückleitungsabschnitt (22) gegenüberliegenden Seite gelagerten Ende des Speicherabschnitts (24, V3) ausgehend zum Ausgangsknoten (S) hin und jeweils zum Basisknoten (NB) hin.

**15.** Elektrische Stromversorgungsschaltung (12m), die speziell für eine elektronische Vorrichtung (1) nach einem der vorhergehenden Ansprüche angeordnet ist.

**16.** Elektronische Vorrichtung zum in-vivo Einbau im menschlichen oder tierischen Körper, ohne Beibehaltung von physischer Verbindung nach Außen, **dadurch gekennzeichnet, dass** sie eine Vorrichtung (1) nach einem der Ansprüche 1 bis 14 umfasst oder bildet oder eine Stromversorgungsschaltung (12m) nach Anspruch 15 umfasst.

**Claims**

**1.** Electronic device (1) of the type operating without an external power supply, comprising an electric power supply circuit (12m) arranged for supplying said device, said electric power supply circuit being arranged for supplying energy for operation of said electronic device via a storage element (13, 24, C2, V3),

said electric power supply circuit (12m) comprising at least one component, called a variable capacitor (11, 211, Cv, Cvar), having an electrical capacitance that varies under the effect of an alternating mechanical movement (10), said variable capacitor

> ◦ being arranged to receive said mechanical movement (10) from the environment of said electronic device (1),
> ◦ and being connected to said storage device (13, 24, C2, V3) by means of a rectifier circuit (23, 23') to increase the electrical energy stored there;

wherein said electric power supply circuit (12m) comprises:

- a generating branch (21) formed by at least the variable capacitor (211, Cv) and a biasing capacitor (212, C1) mounted in series, said generating branch being mounted in parallel with the rectifier circuit (23, 23') and with a storage branch (24) comprising the storage element, between

> ◦ a node, called base node, (NB) that is located at the end of the generating branch (21) on the variable capacitor (211, Cv) side and is maintained at a reference potential,
> ◦ a node, called output node, (S) located at the end of the generating branch (21) on the biasing capacitor (211, C1) side;

- a charge return branch (22) of a type able to conduct a unidirectional electric current:

> ◦ to the generating branch (21), by at least one first end connected to a node (P), called a biasing node, which is located in the generating branch between the variable capacitor (211, Cv, Cvar) and the biasing capacitor (212, C1);
> ◦ from the rectifier (23), by at least one second end receiving a part of the electrical energy produced by said power supply circuit (12m),

wherein the assembly formed by said generating branch (21), the rectifier circuit (23, 23'), the storage branch (24) and the charge return branch (22) constitutes an electric circuit the behaviour of which is passive and remains unchanged and permanent in its characteristics during at least one plurality of cycles of the variable capacitor (211, Cv, Cvar),

thus carrying out at each cycle an additional charge of the biasing capacitor (212, C1) according to an operating mode that is self-synchronizing, i.e. it does not require any means for synchronization with respect to the mechanical movements during said plurality of cycles;

and being **characterized in that** the electric power supply circuit (12m) comprises a circuit arranged to form a voltage multiplier circuit (MT, MT1, MT2, MT3, MT4) that is connected to the generating branch (21) at a biasing node (P) located between the variable capacitor (211, Cv, Cvar) and the biasing capacitor (212, C1), for applying a voltage there that is multiplied relative to the voltage that exists between

- on the one hand the output node (S) of said generating branch (21), and
- on the other hand the base node (NB) or one (OUT) of the ends of the storage branch (24, C2, V3).

2. Device according to the preceding claim, **characterized in that** the charge return branch (22) comprises an oriented diode (D3, D3') from the rectifier (23, 23') to the generating branch (21), or several diodes (D, D3 to D9 Figure 16 to Figure 31) mounted in the same direction, and preferably in series.

3. Device according to any one of the preceding claims, **characterized in that** the electric power supply circuit (12m), or at least one or more of the circuits among the generating branch (21), the storage branch (24), the charge return branch (22) and the rectifier circuit (23, 23'), is made so that it only comprises passive components and non-driven semiconductors, and in particular only diodes and capacitors.

4. Device according to any one of the preceding claims, **characterized in that** the power supply circuit (12m) comprises the following elements, and preferably only these:

- a generating branch (21) comprising the variable capacitor (211, Cv, Cvar) and one or more biasing capacitors (212, C1), and preferably only these latter, said generating branch (21) being connected via its base node (NB) to a reference potential, via a node that is common to the rectifier (23) and to the storage branch (24);
- a rectifier circuit (23) comprising the following components, and preferably only these:

  ∘ a diode (D2), or several diodes mounted in the same direction, from the base node (NB) and to the end (S) of the generating branch (21) located on the biasing capacitor (212, C1) side,
  ∘ a diode (D1), or several diodes mounted in the same direction, from the end (S) of the generating branch (21) located on the biasing capacitor (212, C1) side and to the end (OUT) of the storage branch (24) located on the side opposite the base node (NB);

  - a storage branch (24) comprising a storage element, and preferably only the latter, said storage element comprising a capacitor (C2) or a chemical storage element; and
  - a charge return branch (22) comprising a diode (D3, D3'), or several diodes (D, D3 to D9 Figure 16 to Figure 31) mounted in the same direction, from the end (OUT) of the storage branch located on the side opposite the base node (NB) and to a biasing node (P) of the generating branch (21) located between the variable capacitor (211, Cv) and the biasing capacitor (212, C1).

5. Device according to any one of claims 1 to 3, **characterized in that** the power supply circuit (12m) comprises the following elements, and preferably only these:

  - a generating branch (21) comprising the variable capacitor (211, Cv) and one or more biasing capacitors (C1), and preferably only the latter, said generating branch being connected to earth or to a reference potential via the base node (NB);
  - a rectifier circuit (23) comprising the following components, and preferably only these:

    ∘ a diode (D2'), or several diodes mounted in the same direction, to the base node (NB) and from the end (S) of the generating branch located on the biasing capacitor side,
    ∘ a diode (D1'), or several diodes mounted in the same direction, to the end (S) of the generating branch located on the biasing capacitor side and from the end (OUT) of the storage branch (24) located on the side opposite the base node (NB);

    - a storage branch (24) comprising a storage element, and preferably only these latter, said storage element comprising a capacitor (C2) or a chemical storage element; and
    - a charge return branch (22) comprising a diode (D3'), or several diodes mounted in the same direction, to the end (OUT) of the storage branch located on the side opposite the base node and from a biasing node (P) of the generating branch (21) located between the variable capacitor (211, Cv) and the biasing capacitor (212, C1).

6. Device according to claim 1, **characterized in that** the power supply circuit (12m) comprises an electrical energy storage component having a point with intermediate voltage, forming for example a mid-point, which is connected to the base node (NB) and thus delimits:

  - a first storage part (C2) that forms the storage element of the storage branch; and
  - a second storage part (C3) that is mounted in series in the rectifier circuit (23) between the base node (NB) and a biasing node (P) of the generating branch (21) located between the variable capacitor (211, Cv) and the biasing capacitor (212, C1).

7. Device according to any one of the preceding claims, **characterized in that** the rectifier circuit (23) comprises, in a branch forming a single direction between the base node (NB) and the end (S) of the generating branch (21) located on the biasing capacitor (212, C1) side, a component (K) forming a switch and that is arranged so as to be able to open said branch at the request of a user or in a controlled manner according to a period of time comprising a plurality of cycles of the variable capacitor, and in particular more than 100 or even 1000 cycles.

8. Device according to any one of the preceding claims, **characterized in that** the voltage multiplier circuit is a cascade multiplier of a series type (MT1, MT2, MT3) or parallel type (MT1, MT2, MT3, MT4).

9. Device according to the preceding claim, **characterized in that** the multiplier circuit (MT1) comprises a cascade multiplier that contributes to the function of charge return circuit (22) and that comprises at least a first, a second and a third branching node (M1, M2, M3) and is mounted according to a first configuration so that:

- the first branching node (M1) and the second branching node (M2) between them form a branch able to conduct a unidirectional current, and are connected

  ○ for the one (M1), to a biasing node (P) located in the generating branch (21) between the variable capacitor (211, Cv, Cvar) and the biasing capacitor (212, C1), and
  ○ for the other (M2), to the end (OUT) of the storage branch (24, C2, V3) on the side opposite the base node (NB);

- the third branching node (M3) is connected to the output node (S).

10. Device according to any one of claims 1 to 8, **characterized in that** the multiplier circuit (MT2) comprises a cascade multiplier that contributes to the charge return circuit and that comprises at least a first, a second and a third branching node (M1, M2, M3) and is mounted according to a second configuration so that:

  - the first branching node (M1) and the second branching node (M2) between them form a branch able to conduct a unidirectional current, and are connected

    ○ for the one (M1), to a biasing node (P) located in the generating branch (21) between the variable capacitor (211, Cv, Cvar) and the biasing capacitor (212, C1), and
    ○ for the other (M2), to the output node (S);

  - the third branching node (M3) is connected to the end (OUT) of the storage branch (24, V4) on the side opposite the base node (NB).

11. Device according to any one of claims 1 to 8, **characterized in that** the multiplier circuit (MT3) is a cascade multiplier that comprises at least a first, a second and a third branching node (M1, M2, M3) and is mounted according to a third configuration so that:

  - the first branching node (M1) and the second branching node (M2) between them form a branch able to conduct a unidirectional current, and are connected

    ○ for the one (M1), to a biasing node (P) located in the generating branch (21) between the variable capacitor (211, Cv, Cvar) and the biasing capacitor (212, C1), and
    ○ for the other (M2), to the output node (S);

  - the third branching node (M3) is connected to the base node (NB).

12. Device according to any one of claims 1 to 8, **characterized in that** the multiplier circuit (MT4) is a cascade multiplier that comprises at least a first, a second and a third branching node (M1, M2, M3) and is mounted according to a fourth configuration so that:

  - the first branching node (M1) and the second branching node (M2) between them form a branch able to conduct a unidirectional current, and are connected

    ○ for the one (M1), to a biasing node (P) located in the generating branch (21) between the variable capacitor (211, Cv, Cvar) and the biasing capacitor (212, C1), and
    ○ for the other (M2), to the base node (NB);

  - the third branching node (M3) is connected to the output node (S).

13. Device according to any one of the preceding claims, **characterized in that** the biasing capacitor (212, C1) has an electrical capacitance having a value of at least five times the variation in the capacitance of the variable capacitor (211, Cv, Cvar) during a cycle (10), and/or **in that** the storage element has an electrical energy storage capacity corresponding to a capacitor (C2, V3) the electrical capacitance of which is greater than three times that of the biasing capacitor (212, C1).

14. Device according to any one of the preceding claims, **characterized in that** the rectifier circuit (23') comprises at least one bridge of at least four diodes forming:

- at least two branches (D1 and D7, or D1 and D9) forming a unidirectional connection to the end (OUT) of the storage branch (24) on the side of the charge return branch (22), from the output node (S) and from the base node (NB), respectively; and
- at least two branches (D2 and D8, or D2 and D10) forming a unidirectional connection from the end of the storage branch (24, V3) of the side opposite the charge return branch (22), to the output node (S) and to the base node (NB), respectively.

**15.** Electric power supply circuit (12m) arranged specifically for an electronic device (1) according to any one of the preceding claims.

**16.** Electronic device intended to be implanted *in vivo* in the human body or in the body of an animal without maintaining physical connection with the outside, **characterized in that** it comprises or forms a device (1) according to any one of claims 1 to 14 or comprises a power supply circuit (12m) according to claim 15.

**Fig. 1**
Art antérieur

**Fig. 2**

**Fig. 3**

**Fig. 4**

**Fig. 5a**

**Fig. 5b**

**Fig. 5c**

**Fig. 6a**
phase 1

C1  D3
D1
$C_{var}$  V  V1  D2
C2  Vout

**Fig. 6b**
phase 2

C1  D3
D1
$C_{var}$  V  V1  D2
C2  Vout

**Fig. 6c**
phase 3

C1  D3
D1
$C_{var}$  V  V1  D2
C2  Vout

**Fig. 6d**
phase 4

C1  D3
D1
$C_{var}$  V  V1  D2
C2  Vout

**Fig. 6e**
phase 5

C1  D3
D1
$C_{var}$  V  V1  D2
C2  Vout

**Fig. 6f**

**Fig. 7**

**Fig. 8**

**Fig. 9**

**Fig. 10a**

22  retour de charge

MT

12m

P  polarisation
212

S

OUT

211

redresseur

stockage

NB

23

24

21 génération

**Fig. 10b**
multiplicateur 2 cellules
$V_{c1}=3.V_{out}$

MC2

C4
100p

C2
100p

MC1

D7  D6  D5  D4  D3
D    D    D    D    D

C5
100p

C3
100p

$V_e$

22

cap+  Cvar

C1

S

D1
D

OUT  $V_{out}$

P

1n

cap-

D2
D

C2

NB

NB  NB

**Fig. 11**
multiplicateur type 1

22  M1  MT1  M2

M3

OUT

P  C1  S

D1

Cv

D2

C2

21

NB

23

24

**Fig.12**
multiplicateur type 2

**Fig. 13**
multiplicateur type 3

**Fig.14**
multiplicateur type 4

**Fig. 15**

**Fig. 16**

multiplicateur parallèle x1
type 1
$V_{c1}=V_{out}$

**Fig. 17**

multiplicateur parallèle x1
type 2
$V_{c1}=V_{out}$

**Fig. 18**

multiplicateur parallèle x2
type 1
$V_{c1}=2.V_{out}$

**Fig. 19**

multiplicateur parallèle x2
type 2
$V_{c1}=2.V_{out}$

**Fig. 20**

multiplicateur parallèle x2
type 3
$V_{c1}=2.V_{out}$

**Fig. 21**

multiplicateur parallèle x2
type 4
$V_{c1}=2.V_{out}$

**Fig. 22**

multiplicateur parallèle x3
type 1
$V_{c1}=3.V_{out}$

## Fig. 23

multiplicateur parallèle x3
type 2
$V_{c1} = 3 . V_{out}$

## Fig. 24

multiplicateur parallèle x4
type 1
$V_{c1} = 4 . V_{out}$

## Fig. 25

multiplicateur parallèle x4
type 2
$V_{c1} = 4 . V_{out}$

## Fig. 26

multiplicateur série x2
type 1
$V_{c1} = 2 . V_{out}$

**Fig. 27**

multiplicateur série x2
type 2
$V_{c1}=2.V_{out}$

**Fig. 28**

multiplicateur série x2
type 3
$V_{c1}=2.V_{out}$

**Fig. 29**

multiplicateur série x2
type 4
$V_{c1}=2.V_{out}$

**Fig. 30**

multiplicateur série x3
type 1
$V_{c1}=3.V_{out}$

# Fig. 31

multiplicateur série x3
type 2
$V_{c1} = 3.V_{out}$

# Fig. 32

avec pont complet et
multiplicateur parallèle x4
type 3
$V_{c1} = 4.V_{out}$

# Fig. 33

avec pont complet et
multiplicateur parallèle x3
type 4
$V_{c1} = 3.V_{out}$

## RÉFÉRENCES CITÉES DANS LA DESCRIPTION

**Documents brevets cités dans la description**

- WO 2013059562 A1

**Littérature non-brevet citée dans la description**

- **MENINGER et al.** Vibration-to-Electric Energy Conversion. *IEEE Transactions on Very Large Scale Integration (VLSI) Systems,* 2001, vol. 9, 64-76
- **TORRES et al.** *IEEE International Midwest Symposium on Circuits and Systems, MWSCAS '06,* 2006, 65-69
- **BERNARD C. YEN.** A Variable-Capacitance Vibration-to-Electric Energy Harvester. *IEEE Transactions On Circuits And Systems,* 2005, vol. 53 (2), 288-295